(19) Europäisches Patentamt
European Patent Office
Office européen des brevets

(11) Publication number: **0 211 236**
**A1**

(12) # EUROPEAN PATENT APPLICATION

(21) Application number: 86108983.7

(22) Date of filing: 02.07.86

(51) Int. Cl.⁴: **C 07 D 499/00**
C 07 D 205/08, A 61 K 31/43
//C07F7/18

(30) Priority: 10.07.85 GB 8517450

(43) Date of publication of application:
25.02.87 Bulletin 87/9

(84) Designated Contracting States:
AT BE CH DE FR IT LI LU NL SE

(71) Applicant: HOECHST AKTIENGESELLSCHAFT
Postfach 80 03 20
D-6230 Frankfurt am Main 80(DE)

(72) Inventor: Barker, Andrew John
152 Sutherland Grove West Bletchley
Milton Keynes Bucks(GB)

(72) Inventor: Cooke, Michael David
10 Westbury Lane
Newport Pagnell Bucks(GB)

(72) Inventor: Carruthers, Nicholas Iain
173 Ridge Road Apartment H3
Cedar Grove New Jersey 07009(US)

(54) **7-Oxo-4-thia-1-azabicyclo]3,2,0[hept-2-ene derivatives.**

(57) Compounds of formula I

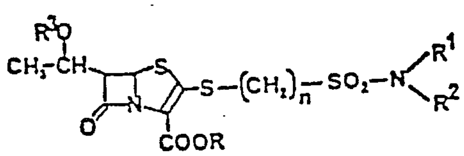

(I)

in which

n represents an integer of from 3 to 6;

R represents a hydrogen atom or a carboxylic acid esterifying group;

$R^1$ and $R^2$, which may be the same or different, each represents an amino protecting group, a hydrogen atom, a straight or branched chain alkyl group having from 1 to 4 carbon atoms, or a cycloalkyl group having from 3 to 6 carbon atoms,

an alkyl or cycloalkyl group being unsubstituted or substituted by an $NH_2$ group which may be protected if desired or required; or

$R^1$ and $R^2$ form an alkylene group having from 3 to 5 carbon atoms; and

$R^3$ represents a hydrogen atom or a hydroxy protecting group;

and salts thereof, have antibacterial and β-lactamase inhibiting activity.

EP 0 211 236 A1

Croydon Printing Company Ltd.

7-OXO-4-THIA-1-AZABICYCLO[3,2,0]HEPT-2-ENE DERIVATIVES

This invention relates to 7-oxo-4-thia-1-azabicyclo-[3,2,0]hept-2-ene derivatives, to a process for their preparation, to pharmaceutical preparations comprising them, and to intermediates for use in the preparation of substances having antibacterial activity and/or β-lactamase inhibitory and/or inactivating activity.

It is well known that penicillin antibiotics have been used extensively for many years to combat bacterial infections in humans and other animals. The first of the penicillin antibiotics to come into general therapeutic use was benzylpenicillin, and this compound still finds widespread use today.

There is, however, a continuing need for new antibiotics, not only to combat bacterial pathogens that do not respond satisfactorily to treatment with conventional penicillins, but also to combat those strains of bacteria which, although once susceptible to penicillin treatment, are now resistant to such therapy. Accordingly, in the search for new types of antibiotics, the discovery of compounds which possess a wide spectrum of activity together with an ability to combat infections caused by penicillin-resistant organisms is an important goal.

The penicillins as a class have in common the β-lactam structure A commonly known as the "penam" nucleus. The introduction of a double bond between carbon atoms 2 and 3 in this structure gives rise to the "penem" nucleus B.

(A) 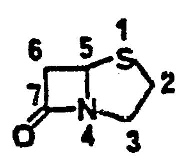

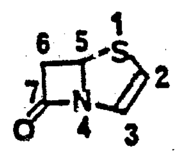 (B)

In recent years considerable interest has been shown in penem antibiotics since they often show high antibiotic activity even against strains of bacteria known to be resistant to penicillin-based therapy. The present invention relates to penem derivatives, but in the present specification the semi-systematic "penam" and "penem"

nomenclature is not used for naming compounds. Instead the β-lactam penem nucleus B is given the systematic name "7-oxo-4-thia-1-azabicyclo[3,2,0]hept-2-ene", with the numbering shown in formula C. A side chain at position 6 is numbered as shown in formula D:

(C) 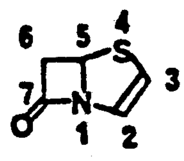

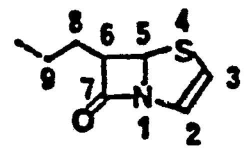 (D)

The present invention provides a compound of formula I

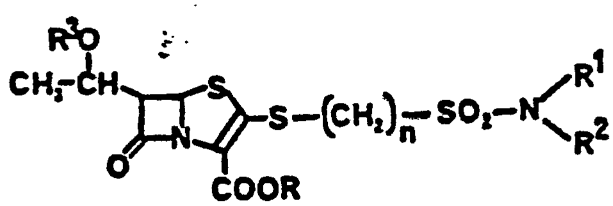 (I)

in which

n represents an integer of from 3 to 6;

R represents a hydrogen atom or a carboxylic acid esterifying group;

$R^1$ and $R^2$, which may be the same or different, each represents an amino protecting group, a hydrogen atom, a straight or branched chain alkyl group having from 1 to 4 carbon atoms, or a cycloalkyl group having from 3 to 6 carbon atoms,

an alkyl or cycloalkyl group being unsubstituted or substituted by an $NH_2$ group which may be protected if desired or required; or

$R^1$ and $R^2$ form an alkylene group having from 3to 5 carbon atoms; and

$R^3$ represents a hydrogen atom or a hydroxy protecting group;

and salts thereof.

Examples of alkyl groups $R^1$ and $R^2$ are methyl, ethyl, <u>n</u>-propyl, <u>iso</u>-propyl, <u>n</u>-butyl, <u>iso</u>-butyl, <u>sec</u>-butyl and <u>t</u>-butyl groups, and examples of cycloalkyl groups are

cyclopentyl and cyclohexyl groups. Such groups may be substituted by an amino group, for example, giving a 2-aminoethyl group, and an amino group may itself be protected. Furthermore, $R^1$ and/or $R^2$ may themselves represent amino-protecting groups. When $R^1$ and $R^2$ form an alkylene group having from 3 to 5 carbon atoms then $R^1$, $R^2$ and the nitrogen atom to which they are attached form a saturated 4 to 6-membered ring, for example, a pyrrolidine or piperidine group.

Preferred compounds of formula I are, for example, those in which $R^1$ and $R^2$ both represent hydrogen atoms, both represent methyl groups, or one represents a hydrogen atom and the other represents a methyl group or a 2-aminoethyl group.

Particularly preferred compounds of formula I are, for example, those in which

(i) n represents the integer 3, and $R^1$ and $R^2$ both represent hydrogen atoms;

(ii) n represents the integer 3, $R^1$ represents a hydrogen atom and $R^2$ represents a methyl group;

(iii) n represents the integer 3, and $R^1$ and $R^2$ both methyl groups; and

(iv) n represents the integer 3, $R^1$ represents a hydrogen atom and $R^2$ represents a 2-aminoethyl group.

Especially preferred are, for example, those compounds of formula I in which n represents the integer 3, and $R^1$ and $R^2$ both represent hydrogen atoms.

In a compound of formula I, a carboxy protecting group R is preferably an ester group that can be converted by hydrolysis, by photolysis, by reduction or, especially, by esterase enzyme action, to give the free acid of formula I. Moreover, in a compound of formula I, a hydroxy protecting group $R^3$ is also preferably a group that can be converted by hydrolysis, by photolysis, by reduction or, especially, by esterase enzyme action, to give a compound of formula I having a free 8-hydroxy group. Details of such carboxy and hydroxy protecting groups are given below.

Groups that can be removed by esterase action are groups that can be cleaved *in vivo*. Compounds having such groups are known as "prodrugs". Preferred compounds of formula I are those in which, independently, R and $R^3$ each represents a hydrogen atom or a physiologically removable group.

The hydroxy group and the carboxy group in a compound of formula I may be esterified or otherwise protected independently of each other.

The present invention provides salts of a compound of formula I, especially physiologically tolerable salts thereof. A salt may be formed at the 2-carboxylic acid group or at any basic centre present. Moreover, when both an acidic centre and a basic centre are present, a compound of formula I may exist in a zwitterionic form.

A compound of formula I may exist in various isomeric forms, all of which are part of the present invention. The stereochemistry at positions 5, 6 and 8 of a compound of formula I can be R or S, independently (R and S being as defined by the Cahn-Ingold-Prelog system of nomenclature). The preferred stereochemistry at position 5 is R, at position 6 is S, and at position 8 is R, and is especially 5R, 6S, 8R.

The present invention also provides a process for the production of a compound of formula I or an ester or salt thereof, which comprises

(i) reacting a compound of formula IIa or its tautomer of formula IIb or a mixture thereof

(IIa) 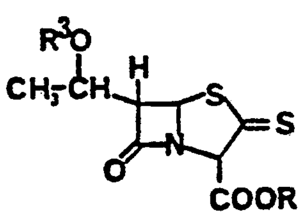 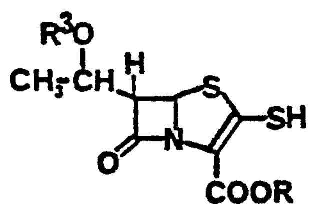 (IIb)

in which R is as defined above and $R^3$ represents a hydrogen atom or a hydroxy protecting group, with a compound of formula III

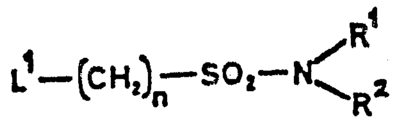

(III)

in which n, $R^1$ and $R^2$ are as defined above, and $L^1$ represents a leaving group; or

(ii) treating a compound of formula IV

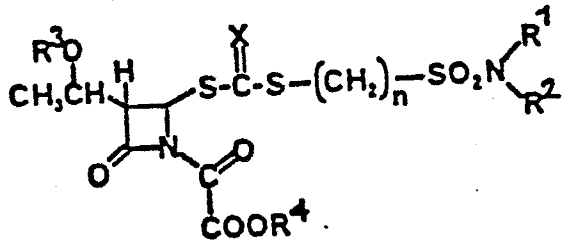

(IV)

in which n, $R^1$ and $R^2$ are defined as above, $R^4$ represents a carboxy protecting group, and X represents an oxygen atom or, preferably, a sulphur atom, with a trivalent organophosphorus reagent to effect cyclisation to give a compound of formula I; or

(iii) reacting a compound of formula V

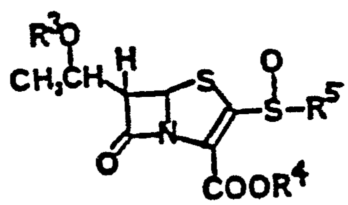

(V)

in which $R^3$ and $R^4$ are defined as above, and $R^5$ represents a straight or branched chain alkyl group having from 1 to 6 carbon atoms which may be substituted by one or more fluorine atoms, or represents an aryl group as hereinafter defined, especially a phenyl group, with a compound of formula VI

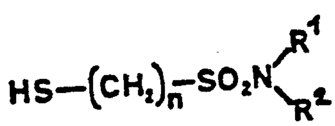

(VI)

in which n, $R^1$ and $R^2$ are defined as above, generally in the presence of a base, or with a reactive derivative of a compound of formula VI and, if desired, carrying out any one or more of the following steps in any desired order:

a) hydrolysing a 2-carboxylic ester of formula I to give the corresponding free acid of formula I,

b) reacting a free acid of formula I or a salt thereof with an agent capable of forming a 2-carboxylic acid ester, for example, with an alcohol, a phenol or a reactive derivative thereof to give a 2-carboxylic acid ester of formula I,

c) carrying out an acid or base catalysed ester interchange on a 2-carboxylic acid ester of formula I to give a different ester of formula I,

d) reacting a free acid of formula I with a base to give a salt at the carboxy group at position 2,

e) reacting a free acid of formula I or a 2-carboxylic acid ester of formula I having a basic group present with an acid to give an acid addition salt thereof,

f) hydrolysing the ester group from a 2-carboxylic acid ester of formula I in the presence of a salt-forming agent, for example, an alkali metal salt, to give a salt of a compound of formula I at the 2-carboxy group,

g) reacting a salt of a compound of formula I with an acid to give a free acid of formula I,

h) reacting a free acid of formula I or a salt thereof, or a 2-carboxylic acid ester of formula I, with an organic acid derivative to give a free acid of formula I or a 2-carboxylic acid ester of formula I having an esterified hydroxy group at the 8-position,

i) removing a carboxylic acid acyl group or a hydroxy protecting group from a modified 8-hydroxy group to give a compound of formula I having a free 8-hydroxy group, and

j) removing any amino protecting groups present.

The term "aryl" is defined in IUPAC Rule A-13.5, Nomenclature of Organic Chemistry (1979), Pergamon Press.

In formula I, $R^3$ may represent a hydrogen atom or a protecting group. In certain of the processes involved in the production of a compound of formula I, it may be preferable to protect the 8-hydroxy group. Hydroxy protecting groups and methods for their introduction and removal are known (that is to say, in actual use in the art or described in the literature of the art) see, for

8. A process as claimed in any one of claims 1 to 7, wherein a hydroxy protecting group $R^3$ is a group that can be converted by hydrolysis, by photolysis, by reduction or by esterase enzyme action to give a compound of formula I having a free 8-hydroxy group, for example, $R^3$ is a carboxylic acid group of the formula $R^{20}CO$- in which $R^{20}$ represents a hydrogen atom or a straight or branched chain alkyl group having from 1 to 6 carbon atoms, or represents a phenyl group or a phenoxyalkyl group in which the alkyl moiety is straight-chained or branched and has from 1 to 4 carbon atoms, for example, $R^{20}$ represents a methyl, ethyl or t-butyl group, or a phenoxymethyl group.

9. A process as claimed in any one of claims 1 to 6, wherein R represents a hydrogen atom or a group that can be cleaved <u>in vivo</u> to give a free carboxy group or a carboxylate group, and $R^3$ represents a hydrogen atom or a group that can be cleaved <u>in vivo</u> to give a free hydroxy group.

10. A process as claimed in any one of claims 1 to 9, according to which there is produced a compound of formula I having, independently or in any combination, 5R-stereochemisty, 6S-stereochemisty and 8R-stereochemistry, especially 5R,6S,8R-stereochemistry.

11. A process as claimed in claim 1, according to which there is produced 5(R),3-(3-dimethylaminosulphonyl-propylthio)-6(S)-[1(R)-hydroxyethyl]-7-oxo-4-thia-1-azabicyclo[3.2.0]hept-2-ene-2-carboxylic acid, or a salt thereof, or an ester thereof at the 2- and/or 8-position.

12. A process as claimed in claim 1, according to which there is produced 5(R),6(S)-[1(R)-hydroxyethyl]-3-(3-methylaminosulphonylpropylthio)-7-oxo-4-thia-1-azabicyclo[3.2.0]hept-2-ene-2-carboxylic acid, or a salt thereof, or an ester thereof at the 2- and/or 8-position.

13. A process as claimed in claim 1, according to which there is produced 5(R),3-(3-aminosulphonylpropylthio)-6(S)-[1(R)-hydroxyethyl]-7-oxo-4-thia-1-azabicyclo[3.2.0]hept-2-ene-2-carboxylic acid, a salt thereof, or an ester thereof at the 2- and/or 8-position.

14. A process for the manufacture of a pharmaceutical preparation which comprises admixing or conjoining a compound of formula I as prepared according to any one of claims 1 to 13, or a physiologically tolerable salt thereof, or a mixture of two or more such substances, with a pharmaceutically suitable carrier.

15. A process for the production of a compound of formula IV, which comprises reacting a compound of formula XIII

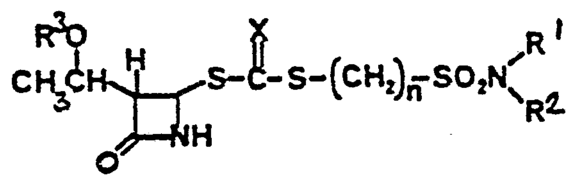

(XIII)

in which n, $R^1$, $R^2$, $R^3$ and X are as defined in claim 1, with an acylating agent of formula XIV

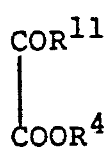

(XIV)

in the range of from -80 to +80°C., preferably from, -40 to +30°C., with room temperature especially preferred.

The reaction is generally carried out in a solvent or diluent that is inert under the reaction conditions, for example, an oxygenated hydrocarbon, for example, tetrahydrofuran, dioxane or ethyl acetate; a chlorinated hydrocarbon, for example, methylene chloride or chloroform; or a polar organic solvent, for example, N,N-dimethylacetamide, N,N-dimethylformamide or acetonitrile; or water. A mixture of two or more solvents may be used, for example, a mixture of water and another solvent (whether miscible with water or not). Preferred solvents are diethyl ether, tetrahydrofuran, dioxane and acetonitrile.

According to process (ii), a compound of formula IV may be treated with a trivalent organophosphorus reagent to effect cyclisation to give a compound of formula I.

In formula IV, any free amino group present in $R^1$ and/or $R^2$ should be protected, and the sulphonamide nitrogen should be protected if it does not already have two substituents other than hydrogen atoms. $R^3$ preferably represents a hydroxy protecting group, especially a silyl ether group.

Examples of trivalent organophosphorus compounds which may be used to effect cyclisation are phosphites and phosphoramides, for example, of the following formulae VII and VIII, and phosphines of formula IX in combination with phosphites of formula VII

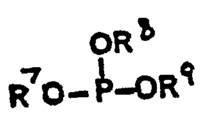

(VII)

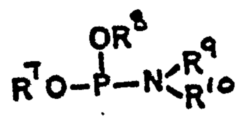

(VIII)

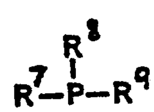

(IX)

in which formulae $R^7$, $R^8$, $R^9$ and $R^{10}$, which may be the same or different, each represents a straight or branched chain alkyl group having from 1 to 6 carbon atoms or a phenyl group which may be unsubstituted or substituted, for example, by a methyl group, especially a para-methyl group;

also cyclic trialkyl phosphites, each alkyl moiety having from 1 to 4 carbon atoms, for example, of formula X

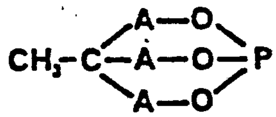

(X)

in which each group A denotes an alkylene group having from 1 to 4 carbon atoms; and also catechol phosphites and catechol dimer phosphites, for example, of the following formulae XI and XII, respectively:

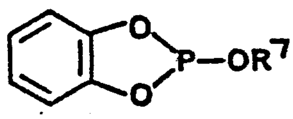

(XI)

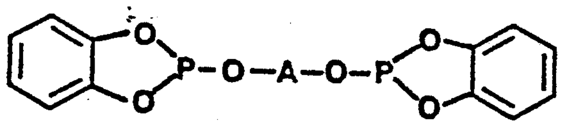

(XII)

in which $R^7$ and A are defined as above. A trivalent organophosphorus compound may be bound to an inert polymer.

A preferred trivalent organophosphorus reagent is a phosphite or phosphoramide of formula VII or VIII respectively, or a combination of a phosphine of formula IX with a phosphite of formula VII, in which formulae the groups $R^7$, $R^8$, $R^9$ and $R^{10}$ are as defined above. Particularly preferred are the trialkyl phosphites, and especially trimethyl phosphite and triethyl phosphite.

When the organophosphorus reagent used to effect cyclisation of compound IV is a phosphite or phosphoramide, generally from 2 to 3 moles thereof are used per mole of compound IV. When a phosphine is chosen, from 1 to 3 moles are generally used per mole of compound IV, in combination with a phosphite, generally 1 mole thereof. The phosphite is preferably added slowly to a mixture of the phosphine and the compound IV.

The cyclisation reaction is generally carried out in a refluxing solvent. When X in compound IV represents a sulphur atom, preferred solvents are aromatic hydrocarbons, for example, benzene, toluene and xylene, and halogenated

hydrocarbons, for example, dichloromethane, chloroform and 1,2-dichloroethane. When X in compound IV represents an oxygen atom, preferred solvents are benzene, toluene and xylene.

A compound of formula IV may be prepared by reacting a compound of formula XIII

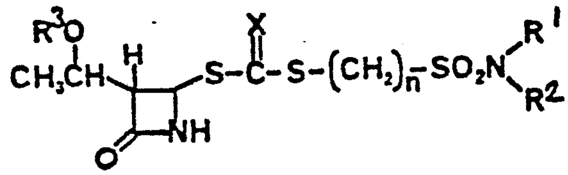

(XIII)

in which n, $R^1$, $R^2$, $R^3$ and X are as defined above, with an acylating agent of formula XIV

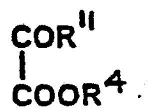

(XIV)

in which $R^4$ is as defined above and $R^{11}$ represents a group that can be displaced by the azetidinone nitrogen in the compound of formula XIII.

In compound XIII, hydroxy and amine groups are preferably protected as described above for compound IV.

Groups $R^{11}$ that can be displaced by the azetidinone nitrogen of compound XIII are known and $R^{11}$ represents, for example, a halogen atom, an imidazolide group, or a mixed anhydride group, for example, a group $-OCOR^{12}$ or $-OC(O)OR^{12}$ in which $R^{12}$ represents a straight or branched chain alkyl group having from 1 to 4 carbon atoms. $R^{11}$ preferably represents a halogen atom, especially a chlorine atom.

The reaction between compound XIII and the acylating agent of formula XIV is carried out in the presence of a base, for example, a tertiary amine, preferably a trialkylamine (each alkyl moiety having from 1 to 4 carbon atoms and having a straight or branched chain, the three alkyl moieties being the same or different), and

especially triethylamine or ethyldiisopropylamine; or pyridine or a substituted pyridine, for example, 4-dimethylaminopyridine. Particularly preferred bases are triethylamine and ethyldiisopropylamine. A mixture of two or more bases may be used.

In addition to an organic base as described above, it is preferable to include an acid binding agent in the reaction mixture. Examples of acid binding agents are alkali metal and alkaline earth metal carbonates and bicarbonates, and organic epoxides, for example, propylene oxide. A preferred binding agent is calcium carbonate.

In general, for each mole of compound XIII there is used from 1 to 2 moles of the acylating agent XIV, from 1 to 4 moles of the amine base (or a total of from 1 to 4 moles of a mixture of amine bases), and from 0 to 10 moles of the acid binding agent.

The compound of formula XIII and the acylating agent of formula XIV are generally reacted in a solvent or diluent that is inert to the reaction, for example, in a halogenated hydrocarbon, for example, dichloromethane or chloroform, in an oxygenated hydrocarbon, for example, an ether, for example, tetrahydrofuran, dioxane or diethyl ether, or in an aromatic hydrocarbon, for example, benzene or toluene. The reaction is generally carried out at a temperature within the range of from -20 to +60°C, preferably from 0 to 20°C.

Compound IV, the product of the acylation reaction, may be isolated if desired, but is generally cyclised without further purification to give a compound of formula I.

A compound of formula XIII may be prepared by reacting a compound of formula XV

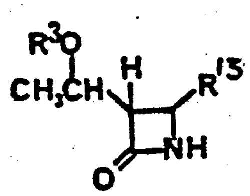

(XV)

in which $R^3$ is as defined above and preferably represents a

hydroxy protecting group, especially a silyl ether group, and $R^{13}$ represents a group that can be replaced in a nucleophilic displacement reaction and is, for example, a halogen atom, especially a chlorine atom; an alkylcarbonyloxy group in which the alkyl moiety has from 1 to 4 carbon atoms, a straight or branched chain, and may be substituted by an electron-withdrawing group, for example, a halogen atom, especially a fluorine atom, for example, a trifluoroacetate group; a phenylcarbonyloxy group, for example a benzoyloxy group; or an $-SO_2R^6$ group in which $R^6$ is as defined above; and $R^{13}$ represents especially an acetoxy group, with a compound of formula XVI

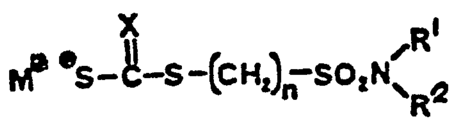

(XVI)

in which X, $R^1$, $R^2$ and n are defined as above, and M represents an alkali metal or alkaline earth metal atom, or an ammonium group that is unsubstituted or substituted, for example, by one to four groups selected from alkyl groups having from 1 to 4 carbon atoms.

Certain compounds of formula XV in which $R^3$ represents a hydroxy protecting group may be prepared as described in Belgian Patent Specification No. 882 764 ($R^3$ represents a dimethyl-t-butylsilyl group), and EPA 0 002 210A ($R^3$ represents a p-nitrobenzyloxycarbonyl group). Other compounds of formula XV may be prepared analogously.

Compound XVI is generally prepared in situ and is not generally isolated before reaction with compound XV.

Compound XVI may be prepared by reacting a compound of formula VI

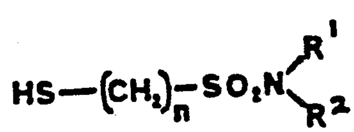

(VI)

in which $R^1$, $R^2$ and n are defined as above, and in which

amino groups are preferably protected as described above for compound IV, with a base, for example, an alkali metal or alkaline earth metal alkoxide having from 1 to 5 carbon atoms, an alkali metal, alkaline earth metal or ammonium hydroxide, an alkali metal or alkaline earth metal hydride, or an alkali metal or alkaline earth metal carbonate, and then with carbon disulphide or carbon oxysulphide (see for example M. Lang, K. Prasad, J. Gosteli, R.B. Woodward, Helv. Chim. Acta., 63, 1093 (1980), and T. Hayashi, A Yoshida, N. Takeda, S. Oida, S. Sugawara, E. Ohki, Chem. Pharm. Bull., 29, 3158 (1981)).

In the above bases, the alkali metal component is preferably sodium or potassium, and preferred bases are sodium hydroxide, potassium hydroxide, sodium methoxide, sodium ethoxide, sodium isopropoxide and potassium t-butoxide, with sodium isopropoxide, sodium hydroxide and potassium hydroxide being particularly preferred.

When the base chosen is a hydride, a solvent that is inert under the reaction conditions may be used, for example, tetrahydrofuran, acetonitrile or diethyl ether; when an alkoxide is chosen as the base, the solvent for the reaction is preferably the corresponding alcohol, or is an inert solvent, for example, tetrahydrofuran or diethyl ether; and when a hydroxide is chosen, a preferred solvent is an alcohol, especially ethanol or a mixture of ethanol and water.

A compound of formula I may be produced as described in (iii) above by reacting a compound of formula V with a compound of formula VI or with a reactive derivative thereof, for example, an alkali metal salt thereof, for example, a sodium salt. The displacement of the sulphinyl group at position 3 of compound V by a compound of formula VI or a reactive derivative thereof is usually carried out in a solvent or diluent that is inert under the reaction conditions, for example, acetone, acetonitrile, dimethylformamide; an oxygenated hydrocarbon, for example, diethyl ether, ethyl acetate, dioxane or tetrahydrofuran; a chlorinated hydrocarbon, for example, dichloromethane or

chloroform; or an aromatic hydrocarbon, for example, benzene or toluene. The reaction temperature is generally within the range of from -60 to +40°C, and is preferably from -5 to room temperature.

The reaction between compounds V and VI generally requires the presence of a base, unless compound VI is already present as a reactive derivative, for example, as an alkali metal salt, in which case a base is not generally necessary. The base used may be organic, for example, a tertiary amine base, for example, a trialkylamine (each alkyl moiety having from 1 to 4 carbon atoms and having a straight or branched chain, the three alkyl moieties being the same or different), especially triethylamine or ethyldiisopropylamine, or pyridine or a substitued pyridine, for example, 4-dimethylaminopyridine, or an inorganic base, for example, an alkali metal alkoxide, for example, sodium methoxide, or an alkali metal hydroxide, for example, sodium hydroxide. Particularly preferred bases are triethylamine and ethyldiisopropylamine.

A compound of formula V may be prepared by oxidising the corresponding compound of formula XVII

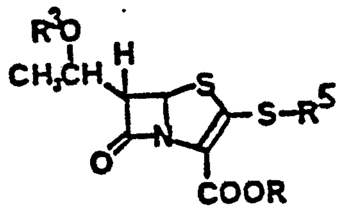

(XVII)

in which $R^3$, $R^4$ and $R^5$ are as defined above, and $R^5$ preferably represents an ethyl group.

The oxidation may be carried out by means of a chemical oxidising agent, for example, a peracid, for example, peracetic acid or m-chloroperbenzoic acid; hydrogen peroxide; an alkylhydroperoxide, for example, t-butyl hydroperoxide; a permanganate, for example, potassium permanganate; a perborate, for example, sodium perborate; or a persulphate, for example, potassium persulphate. Generally from 1 to 3 moles of the oxidising agent are used per mole of compound XVII, and preferably

just over 1 mole of oxidising agent.

The oxidation is carried out in a solvent or diluent that is inert under the reaction conditions, for example, a chlorinated hydrocarbon, for example, dichloromethane or chloroform or an oxygenated hydrocarbon, for example, diethyl ether, tetrahydrofuran or ethyl acetate. Particularly preferred solvents are dichloromethane and ethyl acetate.

The oxidation is generally carried out at a temperature within the range of from -70 to +20°C, preferably from -40 to 0°C.

Compound V is obtained as a mixture of isomers at the oxidised sulphur atom, but separation of the isomers is not necessary as both isomers give the same product when compound V is converted into compound I.

Compounds of formula XVII may be prepared by a process analogous to that described in process (ii) above for the cyclisation of compound IV, or by reacting a compound of formula II with an agent capable of introducing a group $R^5$ as defined above at the thiolactone sulphur atom, for example, an alkylating group of formula XVIII

$$L^1—R^5 \qquad (XVIII)$$

in which $L^1$ and $R^5$ are defined as above. The reaction is carried out analogously to the reaction between compound II and compound III described above.

As mentioned above, a compound of formula XVII may be produced by a process analogous to that described in (ii) above, that is to say, by treating a compound of formula XIX

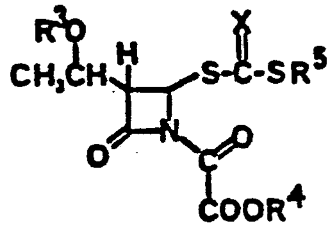

(XIX)

in which X, $R^3$, $R^4$ and $R^5$ are as defined above, with a trivalent organophosphorus reagent to effect cyclisation to compound XVII. The trivalent organophosphorus reagent and the reaction conditions are preferably as described above for the cyclisation of compound IV to give compound I.

Compounds of formula XVII in which $R^5$ represents a phenyl group or a highly fluorinated alkyl group are preferably produced by the cyclisation reaction described above. Other compounds of formula XVII may generally be produced by either the cyclisation process or the alkylation process described above.

A compound of formula XIX may be prepared analogously to compound IV, that is to say, by acylating a compound of formula XX

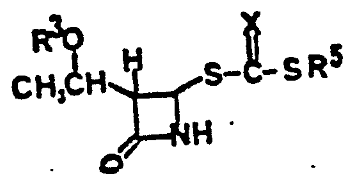

(XX)

in which X, $R^3$ and $R^5$ are as defined above with an acylating agent of formula XIV

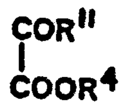

(XIV)

in which $R^4$ and $R^{11}$ are as defined above.

A compound of formula XX may be produced by reacting a compound of formula XV

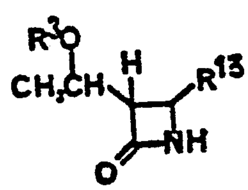

(XV)

in which $R^3$ and $R^{13}$ are as defined above,

with a compound of formula XXI

$$M^{\oplus\ominus}S - \overset{\overset{\displaystyle X}{\|}}{C} - S - R^5 \qquad \text{(XXI)}$$

in which M, X and $R^5$ are as defined above.

Compound XXI may be produced analogously to compound XVI, and the reaction between compounds XX and XIV, and between compounds XV and XXI are preferably carried out analogously to the reactions between compounds XIII and XIV, and compounds XV and XVI, respectively.

Compounds of formula III and VI may be produced by processes analogous to those known for the production of similar compounds, for example, to produce a compound of formula III in which $L^1$ represents an iodine atom, a compound of formula XXII

$$Cl - (CH_2)_n - SO_2Cl \qquad \text{(XXII)}$$

in which n is as defined above, is reacted with the appropriate amine of formula XXIII

$$HNR^1R^2 \qquad \text{(XXIII)}$$

in which $R^1$ and $R^2$ are as defined above to give a compound of formula XXIV

$$Cl - (CH_2)_n - SO_2N\begin{matrix} \diagup R^1 \\ \diagdown R^2 \end{matrix} \qquad \text{(XXIV)}$$

Compound XXIV is then reacted with an iodide, for example, an alkali metal iodide, especially sodium iodide, to give the desired compound of formula XXV

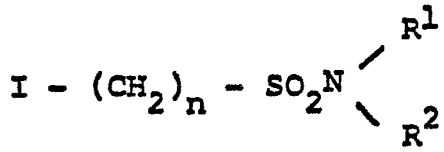

(XXV)

in which n, $R^1$ and $R^2$ are as defined above.

The reaction between compounds XXII and XXIII is generally carried out in an inert solvent, for example, an ether or a chlorinated hydrocarbon, for example, diethyl ether, tetrahydrofuran, dichloromethane or chloroform. The reaction is generally carried out at a temperature in the range of from -20 to +20°C, for example, from -5 to 0°C. The reaction between compound XXIV and the iodide is generally carried out in a solvent, for example, acetone, methyl ethyl ketone or acetonitrile, and under reflux.

Other compounds of formula III may be prepared analogously from a compound of formula XXII, or from a corresponding alcohol or other derivative.

A compound of formula VI may be prepared by reacting a compound of formula XXIV with thiourea to give a compound of formula XXVI

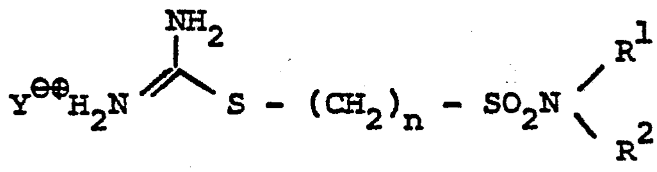

(XXVI)

in which n is as defined above and Y represents a counter-ion, for example, chlorine. The reaction is generally carried out in a solvent, preferably water, and under reflux. The resulting compound of formula XXVI is then treated with a base, preferably an alkali metal hydroxide, especially sodium hydroxide, in a refluxing solvent, especially in aqueous ethanol to give compound VI.

As mentioned above, amino and hydroxy groups may be protected in the intermediates used in the production of a compound of formula I. If an amino group is present as a substituent of an alkyl or cycloalkyl group $R^1$ and/or $R^2$, it should generally be protected. If $R^1$ and/or $R^2$

represents a hydrogen atom, then the sulphonamide group should generally be protected in compound IV and compound XIII, but in other sulphonamide-containing compounds, such protection is not generally necessary, but can be carried out, if desired.

Amino-protecting groups are well known, as mentioned above, and include, for example, alkyl- and aryloxycarbonyl groups, for example, p-nitrobenzyloxycarbonyl and t-butyloxycarbonyl groups; aralkyl groups, especially benzyl and substituted benzyl groups, for example, p-nitrobenzyl and p-methoxybenzyl groups; alkylcarbonyl groups, for example, trifluoromethylcarbonyl groups, and silylated groups, for example, as described below in relation to hydroxy protecting groups.

In processes (i) and (iii), it is not generally necessary to protect the 8-hydroxy group. In process (ii) and in the reactions involved in the production of compound IV, however, it is generally preferable to protect the 8-hydroxy group during the formation of compound I, and to remove the protecting group as one of the last steps in the formation of compound I. Accordingly, in compounds of formulae IV, XIII and XV $R^3$ preferably represents a hydroxy protecting group. When compound XVII is produced by the acylation/cyclisation route, then the 8-hydroxy group is preferably protected during these steps, that is to say, $R^3$ in compounds XV, XX and XIX is preferably a protected hydroxy group, but $R^3$ in compound V may be either free or protected.

As mentioned above, hydroxy protecting groups and methods for their introduction and removal are well known see, for example, McOmie <u>loc sit</u> and Greene <u>loc sit</u>.

Particularly useful as hydroxy protecting groups $R^3$ are those that can be removed under acidic conditions. Such groups are well known in the art and are, for example, tetrahydropyranyl and tetrahydrofuranyl groups, and acetal and ketal groups, for example, of formula

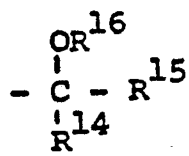

in which $R^{14}$ and $R^{15}$, which may be the same or different, each represents a hydrogen atom or an alkyl group having from 1 to 4 carbon atoms, preferably a methyl group, or $R^{14}$ and $R^{15}$ together with the carbon atoms to which they are attached, represent a cycloalkyl ring having from 4 to 7 carbon atoms, and $R^{16}$ represents an alkyl group having from 1 to 4 carbon atoms, preferably a methyl or ethyl group, or $R^{14}$ and $R^{16}$, together with the carbon atom and the oxygen atom to which they are attached, respectively, represent a tetrahydropyranyl ring.

Particularly useful are silyl ethers, for example, having three substituents on the silicon atom, and preferably up to 24 carbon atoms in total, the three substituents being the same or different, and selected from alkyl, alkenyl and cycloalkyl groups, and phenyl and phenalkyl groups which may be unsubstituted or substituted as defined above, for example, $-SiR^{17}R^{18}R^{19}$ groups, in which $R^{17}$, $R^{18}$ and $R^{19}$, are the same or different, and each represents a lower alkyl group or a phenyl group, for example, giving trimethylsilyl, triethylsilyl, diphenyl-*t*-butylsilyl, dimethyl-*t*-butylsilyl, and methyldiphenylsilyl groups.

Further examples of hydroxy protecting groups are carbonate derivatives, for example, 2,2,2-trichloroethoxycarbonyl groups, and *p*-nitrobenzyloxycarbonyl groups.

Preferred $R^3$ groups are tetrahydropyranyl, 2-methoxyprop-2-yl, trimethylsilyl, triethylsilyl and, especially, t-butyldimethylsilyl groups.

Such groups may be removed by acid hydrolysis, for example, using 0.1 to 2M, preferably 0.5M hydrochloric acid, for example, 6M HCl in, for example, tetrahydrofuran, cf. Belgian Patent Specification No. 881 012; n-$Bu_4NF$ in an acidic medium, for example, in acetic acid, cf. Belgian Patent Specification No. 882 764; or aqueous hydrogen fluoride, for example, in the presence of acetonitrile, cf. J. Chem. Soc. Perkin 1, 1981, 2055.

In a compound of formula I, the 8-hydroxy group, if esterified, is preferably esterified with a group that can be removed in vivo to give the free hydroxy group, that is to say, an ester group that can be removed under physiological conditions. Examples of suitable esterifying groups are carboxylic acid acyl groups of the formula $R^{20}CO$- in which $R^{20}$ represents a hydrogen atom or a straight or branched chain alkyl group having from 1 to 6 carbon atoms, especially a methyl, ethyl or t-butyl group, or represents a phenyl group or a phenoxyalkyl group in which the alkyl moiety is straight-chained or branched and has from 1 to 4 carbon atoms, and is especially a methylene group.

A non-physiologically removable protecting group $R^3$ is generally removed from a resulting compound of formula I, and may be replaced by a physiologically removable group, if desired. In some cases, a carboxylic acid acyl group $R^3$ may fulfil a protective role during the synthesis of compound I. Such a dual function protective group may be removed, retained or replaced in formula I, as desired.

An ester group at the 8-position may be the only ester group present, or it may be present in addition to an ester group at the 2-carboxyl group. As mentioned above, a physiologically removable group may be present as a protecting group $R^3$ during the formation of a compound of formula I, or it may be introduced at the free 8-hydroxy

group of a compound of formula I, after removal of a non-physiologically removable hydroxy protecting group, if present. (A free amino group present as a substituent of an alkyl or cycloalkyl group $R_1$ and/or $R_2$ should be protected before esterification of the 8-hydroxy group.) An esterifying group may be introduced at the 8-hydroxy group by a reaction with an organic acid derivative in known manner. A particularly convenient method is to react a compound of formula I with an activated acid derivative, for example, an acid anhydride in the presence of an organic base, for example, 4-dimethylaminopyridine.

As indicated above, a compound of formula I may be in the form of an ester at the carboxy group at position 2. Such an ester is particularly one that can be converted into the free acid by hydrolysis, photolysis, reduction or esterase enzyme action. Examples of such esters are those formed with unsubstituted or substituted aliphatic alcohols or phenols having up to 20 carbon atoms in total. In an esterified carboxy group -COOR, the group R may be, for example, a straight or branched chain substituted or unsubstituted alkyl, alkenyl or alkynyl group having up to 18 carbon atoms, preferably up to 8 carbon atoms, and especially up to 6 carbon atoms, for example, a methyl, ethyl, n-propyl, iso-propyl, n-butyl, sec-butyl, iso-butyl, tert-butyl, n-pentyl, n-hexyl, allyl or vinyl group. An aliphatic group R, especially a methyl or ethyl group, may be substituted, for example, by an acyloxy group (further details of such groups are given below); by an aminoalkanoyloxy group; by an optionally substituted amino group; or, in the case of a methyl group, by one or more unsubstituted or substituted phenyl groups. A phenyl group, either as a phenol or as a substituent of a methyl group, may be substituted, for example, by one or more substituents, selected especially from methoxy and nitro groups and halogen atoms. Examples of phenyl

substituted-aliphatic groups, are benzyl, p-nitrobenzyl, p-methoxybenzyl, benzhydryl and trityl groups.

As indicated above, an ester group is especially one that can be removed by hydrolysis, photolysis, oxidation, reduction or enzyme action, or two or more of these methods may be used, for example, reduction followed by hydrolysis. A group R that can be removed readily without substantial degradation of the rest of the molecule is particularly useful as a carboxy protecting group $R^4$. Examples of esters that are readily split by reduction are phenyl substituted-methyl esters, which may be unsubstituted or substituted, for example, benzyl, p-nitrobenzyl, benzhydryl and trityl esters.

Reduction of an ester, for example, a phenyl substituted-methyl ester, for example, a p-nitrobenzyl ester, may be carried out using hydrogen and a metal catalyst, for example, a noble metal catalyst, for example, platinum, palladium or rhodium, which catalyst may be supported, for example, on charcoal or kieselguhr. Such reductions may be carried out in the presence of a salt forming agent, for example, sodium or potassium bicarbonate, if it is desired to form directly a salt at the 2-carboxylic acid group of formula I.

Alternatively, a p-nitrobenzyl ester may be converted into the corresponding free acid by a two-step method, with an initial reduction of the nitro group followed by hydrolysis. The nitro group may be reduced by noble metal catalysed hydrogenation, for example, using platinum, or palladium on carbon, or by a metal reducing agent, for example, zinc in acetic acid. Other metal reducing agents are, for example, aluminium amalgam, and iron and ammonium chloride, see for example, British Patent Specification No. 1,582,960. Reduction of the nitro group is followed by hydrolysis which may occur in situ during reduction of the nitro group or which may be carried out subsequently by treatment with an acid or a base.

An o-nitrobenzyl ester may be converted into the corresponding free acid by photolysis.

Certain ester groups may be split off by base hydrolysis, for example, acetylmethyl and acetoxymethyl

ester groups.

Other cleavable esters are, for example, silyl esters, for example, trialkylsilyl and trialkylsilylalkyl esters having 1 to 4 carbon atoms in each alkyl moiety, for example, trimethylsilyl and trimethylsilylethyl groups.

There may be used an esterifying group that is removable under physiological conditions, that is to say, the esterifying group is split off in vivo to give the free acid or a carboxylate, for example, an acyloxymethyl or acyloxyethyl ester having from 2 to 12 carbon atoms in the acyl moiety, for example, an acetoxymethyl, 1'-(acetoxy)ethyl or pivaloyloxymethyl ester, a 5-methyldioxalen-2-on-4-yl-methyl ester, an aminoalkanoyloxymethyl ester having from 2 to 12 carbon atoms in the alkanoyl moiety, for example, a glycyloxymethyl, L-valinyloxymethyl or L-leucyloxymethyl ester, or a phthalidyl ester, or a 1'-(alkoxycarbonyloxy)ethyl ester, for example, a 1'-(methoxycarbonyloxy)ethyl or 1'-(ethoxycarbonyloxy)ethyl ester, or an optionally substituted 2-aminoethyl ester, for example, a 2-diethylaminoethyl or 2-(1-morpholino)-ethyl ester.

Preferred esters are the p-nitrobenzyl, phthalidyl, pivaloyloxymethyl, ethoxycarbonyloxymethyl, 5-methyldioxalen-2-on-4-yl-methyl, acetylmethyl, acetoxymethyl, 1'-(acetoxy)ethyl, 1'-(acetyl)ethyl and 1'-(ethoxycarbonyloxy)ethyl esters.

An ester of a compound of formula I, or of any other free acid described herein, may be prepared by reaction of the appropriate free acid with an alcohol, a phenol or a reactive derivative thereof. The reaction is preferably carried out under mild conditions in order to prevent rupture of the ring system, for example, under neutral or mild acidic or basic conditions, and at temperatures within the range of from -70 to +35°C.

An ester derived from an alcohol may also be produced by reaction of a reactive derivative of the alcohol, for example, a halide, for example, a chloride, bromide or iodide, or hydrocarbonsulphonyl derivative, for example, a

mesyl or tosyl ester, with a salt of an acid of formula I or of another free acid described herein, for example, an alkali or alkaline earth metal salt, for example, a lithium, sodium, potassium, calcium or barium salt, or an amine salt, for example, a triethylammonium salt. The reaction is preferably carried out in a substituted sulphoxide or amide solvent, for example, in dimethylsulphoxide, dimethylformamide, or hexamethylphosphoramide or, alternatively, an ester may be prepared by reaction of the acid with the alcohol or phenol in the presence of a condensing agent, for example, dicyclohexylcarbodiimide.

The present invention also provides salts of those compounds of formula I that have salt-forming groups, especially the salts of a free acid of formula I and acid addition salts of compounds of formula I having a basic group. The salts are especially physiologically tolerable salts, for example, alkali metal and alkaline earth metal salts, for example, sodium, potassium, lithium, calcium, and magnesium salts, ammonium salts, and salts with organic amines; also physiologically tolerable acid addition salts. These may be formed with a suitable inorganic or organic acid, for example, hydrochloric acid, sulphuric acid, or an organic carboxylic or organic sulphonic acid, for example, p-toluene-sulphonic acid.

A salt of a free acid of formula I may be produced by reacting the free acid with the appropriate base in a solvent, preferably under those conditions under which the salt precipitates. A preferred base is potassium 2-ethylhexanoate.

A salt may be produced directly from an ester by splitting off the ester group under suitable reaction conditions, for example, catalytic reduction of an ester, for example, a p-nitrobenzyl ester, in an aqueous/organic solvent, for example, comprising water and ethyl acetate, dioxane or tetrahydrofuran, in the presence of a metal salt, especially a metal bicarbonate, for example, in an equivalent amount or in a slight excess, yields the salt

directly.

When an acidic centre and a basic centre are both present in a compound of formula I, the compound may exist in zwitterionic form.

Protecting groups may be introduced or removed at any appropriate point in the reactions involved in the production of a compound of formula I.

At any stage in the production of a compound of formula I, a compound produced may be isolated from the reaction mixture in which it was prepared and, if desired, purified by the appropriate techniques used for the purification of organic compounds, for example, chromatography and crystallisation.

As indicated above, various intermediates may be produced in the form of mixtures of isomers of various kinds. Such mixtures may be separated or resolved at any stage, or an isomeric mixture may be used per se for subsequent reactions.

A compound of formula I may have the R or S stereochemistry independently at positions 5, 6 and 8. Any mixture of two or more isomeric forms may be resolved, if desired, or a compound of formula I can be used in the form of an isomeric mixture. The preferred stereochemistry at position 5 in compound I is generally R, corresponding to that in naturally occurring penicillins and cephalosporins, at position 6 is S and at position 8 is R.

Compounds of formula I possess excellent activity against gram positive bacteria and gram negative bacteria including the clinically important strains of Staphylococcus aureus that are resistant to methicillin.

The antimicrobial activity was determined by measuring the Minimum Inhibitory Concentration (MIC). The MIC of the compounds was determined by a standard test, the "agar dilution test" according to Lorian (Antibiotics in Laboratory Medicine, Williams and Wilkins, Baltimore/London 1980) as follows:

A two-fold decreasing concentration series of each

compound was prepared in Petri dishes containing 15 ml of Mueller Hinton agar (Difco). One Petri dish containing only the Mueller Hinton agar served as control for bacterial growth. Each Petri dish was inoculated with a multi-point inoculator (Denley), which transfered 0.6 µl of a 1:100 diluted 18 hours culture of the appropriate test bacterium. After 16 to 18 hours of incubation at 37°C, the Petri dishes were examined for growth of bacteria. The lowest concentration of the compound which causes complete inhibition of growth is taken as the MIC.

When assessed by the agar dilution test described above, compounds of formula I are active against gram positive organisms, for example, Staphylococcus aureus and Streptococcus pyogenes and gram negative organisms, for example, Escherichia coli, Enterobacter cloacae, Proteus morganii and Klebsiella sp at test levels of from 0.01 to 100 µg/ml. Moreover, the compounds of formula I and salts thereof show activity against these organisms in the presence of β-lactamase enzymes produced by both gram positive organisms, for example, Staphylococcus aureus and gram negative organisms, for example, Enterobacter cloacae, thus indicating resistance to these enzymes.

Compounds of formula I are also inhibitors of β -lactamase enzymes.

The compounds of formula I and physiologically tolerable salts thereof may be used in humans and other animals to treat, for example, bacterial infections caused by both gram positive and gram negative bacteria, for example, Staphylococcus aureus, Streptococcus pyogenes, Escherichia coli, Bacillus subtilis and Proteus morganii, some strains of which are resistant to conventional penicillin therapy.

The present invention accordingly provides a pharmaceutical preparation which comprises a compound of formula I, or a physiologically tolerable salt thereof, or a mixture of two or more such substances as active ingredient, in admixture or conjunction with a

pharmaceutically suitable carrier. The preparation may also comprise one or more other pharmaceutically active substances, for example another antibacterial substance, especially one having a β-lactam ring. The preparations may be in a form suitable for enteral or parenteral administration, for example, for oral, intravenous or intramuscular administration, for example, as tablets, capsules, syrups, or sterile injectable or infusion solutions. The preparations are advantageously in unit dosage form and preferably comprise from 10 to 2000 mg of the active ingredient per unit dose. The daily dosage of the active ingredient is generally from 20 to 8000 mg, in divided doses, generally up to 4 doses.

The invention also provides the use of a compound of formula I or a physiologically tolerable ester or salt thereof for the manufacture of a medicament for the treatment of bacterial infections.

The invention further provides a method of treating mammals, especially humans, to combat a bacterial infection, which comprises administering to the mammal a therapeutically effective amount of a compound of formula I or a physiologically tolerable ester or salt thereof.

The invention further provides a pharmaceutical preparation which comprises an active ingredient as defined above, in unit dosage form.

The invention also provides a pharmaceutical preparation which comprises an active ingredient as defined above, or a physiologically tolerable salt thereof or a mixture of two or more such substances, and one or more further pharmaceutically active substances, in unit dosage form. Unit dosages are preferably as described above.

The following are examples of preferred compounds of formula I:

(1) 5(R),3-(3-Dimethylaminosulphonyl-propylthio)-6(S)-[1(R)-hydroxyethyl]-7-oxo-4-thia-1-azabicyclo[3.2.0]hept-2-ene-2-carboxylic acid;

(2) 5(R),6(S)-[1(R)-hydroxyethyl]-3-(3-methylamino-

sulphonyl-propylthio)-7-oxo-4-thia-1-azabicyclo[3.2.0]hept-2-ene-2-carboxylic acid; and
(3) 5(R),3-(3-aminosulphonyl-propylthio)-6(S)-[1(R)-hydroxyethyl]-7-oxo-4-thia-1-azabicyclo[3.2.0]hept-2-ene-2-carboxylic acid.

Of the above compounds (1) to (3), the 3-methylaminosulphonyl compound ie compound (2) is preferred, and the 3-aminosulphonyl compound ie compound (3) is particularly preferred.

The above compounds (1) to (3) may also preferably be in the form of a physiologically tolerable salt and/or a metabolisable ester at the 2-carboxylic acid group and/or at the 8-hydroxy group.

The present invention also provides compounds of formulae IV and XIII.

The following Examples illustrate the invention, but are not limiting.

Example 1

3-Chloropropanesulphonamide

Anhydrous gaseous ammonia was bubbled through a cool ($0^{o}C$), stirred solution of 5g of 3-chloropropanesulphonyl chloride in 50ml of dry diethyl ether. The exothermic reaction was complete in approximately 10 minutes after which a further 50ml of diethyl ether was added and the mixture washed with water. The organic layer was separated and the aqueous layer extracted with a further portion of ether. The combined organic layers were washed with water, dried over anhydrous magnesium sulphate and evaporated *in vacuo* to leave the title compound as a white solid (3.2g), m.p. 64-66$^{o}$C.

$\gamma_{max}$ (Nujol* mull) 3360,3250 $cm^{1}$

$\delta$ ($d_6$-acetone) 6.19 (2H,br.s); 3.78 (2H,t,J=7Hz); 3.25 (2H,dd,J=8Hz and 5.5Hz); 2.57-2.00 (2H,m).

* "Nujol" is a Trade Mark.

Example 2

3-Iodopropanesulphonamide

A solution of 5.94g of sodium iodide and 3.12g of 3-chloropropanesulphonamide in 10ml of dry acetone was heated at reflux for 8 hours. After cooling to room temperature the mixture was partitioned between ethyl acetate and water. The aqueous layer was extracted with a further portion of ethyl acetate and the combined organic extracts washed with aqueous sodium metabisulphite solution, dried over anhydrous magnesium sulphate, and evaporated *in vacuo* to give 3.88g of the title compound, m.p. 88-92$^{o}$C.

$\gamma_{max}$ (Nujol mull) 3330,3235 $cm^{-1}$

δ ($d_6$-acetone) 6.08 (2H,br.s); 3.39 (2H,t,J=7Hz); 3.19 (2H,dd,J=8.0Hz and 5Hz); 2.58-2.01 (2H,m).

Example 3

4-Nitrobenzyl 5(R),3-(3-aminosulphonyl-propylthio)-6(S)-[1(R)-hydroxyethyl]-7-oxo-4-thia-1-azabicyclo[3.2.0]hept-2-ene-2-carboxylate.

To a stirred solution of 30.2g of 4-nitrobenzyl 5(R),6(S)-[1(R)-hydroxyethyl]-7-oxo-4-thia-3-thioxo-1-azabicyclo[3.2.0]heptane-2-carboxylate in 250ml of dry tetrahydrofuran was added 19.7g of 3-iodopropanesulphonamide followed by 30.2ml of diisopropylethylamine. The mixture was stirred at room temperature for 3 hours or until t.l.c. analysis indicated reaction was complete and the bulk of the solvents evaporated *in vacuo* . The residue was taken up in ethyl acetate and the organic solution washed with water, then brine and dried over anhydrous magnesium sulphate. Evaporation of the filtered solution left a residue which was chromatographed on silica gel using hexane/ethyl acetate mixtures as eluant to give 27.8g of the title compound, m.p. 148-150°C.

$\gamma_{max}$ (KBr disc) 1768$cm^{-1}$

δ ($d_6$-acetone) 8.24,7.77 (4H,AA'BB',J=8.9Hz); 6.20 (2H,br.s); 5.81 (1H,d,J=1.5Hz); 5.51,5.28 (2H,ABq,J=14.1Hz); 4.30-4.11 (1H,m); 3.85 (1H,dd,J=6.3Hz and 1.5Hz); 3.34-3.07 (4H,m); 2.36-2.18 (2H,m); 1.29 (3H,d,J=6.3Hz).

Example 4

Potassium 5(R),3-(3-aminosulphonyl-propylthio)-6(S)-[1(R)-hydroxyethyl]-7-oxo-4-thia-1-azabicyclo[3.2.0]-hept-2-ene-2-carboxylate.

A mixture of 10.0g of 4-nitrobenzyl 5(R),3-(3-aminosulphonyl-propylthio)-6(S)-[1(R)-hydroxyethyl]-7-oxo-4-thia-1-azabicyclo[3.2.0]hept-2-ene-2-carboxylate in 100ml of ethyl acetate, 1.99g of potassium bicarbonate in 100ml of water and 10.0g of 10% palladium on charcoal catalyst was hydrogenated at about 350 kPa (about 50 p.s.i.) of hydrogen in a Parr apparatus for 1 hour. The mixture was filtered through "Hyflo" (Trademark) and the aqueous layer separated, washed with ethyl acetate and lyophilised to give 6.44g of the title compound as a pale yellow solid.

$\gamma_{max}$ (KBr disc) 1769 $cm^{-1}$

δ ($D_2O$) 5.68 (1H,d,J=1.4Hz); 4.31-4.15 (1H,m); 3.91 (1H,dd,J=5.9Hz and 1.4Hz); 3.38 (2H,m); 3.27-2.88 (2H,m); 2.30-2.13 (2H,m); 1.29 (3H,d,J=5.6Hz).

Example 5

5(R),3-(3-aminosulphonyl-propylthio)-6(S)-[1(R)-hydroxyethyl]-7-oxo-4-thia-1-azabicyclo[3.2.0]hept-2-ene-2-carboxylic acid.

A solution of 350mg of potassium 5(R),3-(3-aminosulphonyl-propylthio)-6(S)-[1(R)-hydroxyethyl]-7-oxo-4-thia-1-azabicyclo[3.2.0]hept-2-ene-2-carboxylate in 20ml of water was chromatographed on a reverse phase H.P.L.C. column packed with Spherisorb * ODS. Elution with acetonitrile/water mixtures containing traces of formic acid afforded eluant fractions containing the desired acid. The required fractions were collected, frozen and lyophilised to leave 187 mg of the title

compound as a pale yellow solid.

δ ($D_2O$) 5.68 (1H,d,J=1.4Hz); 4.32-4.18 (1H,m); 3.92 (1H,dd,J=6.0Hz and 1.4Hz); 3.44-3.30 (2H,m); 3.21-2.91 (2H,m); 2.30-2.11 (2H,m); 1.30 (3H,d,J=6.4Hz).

* "Spherisorb" is a Trade Mark.

Example 6

Sodium 5(R),3-(3-aminosulphonyl-propylthio)-6(S)-[1(R)-hydroxyethyl]-7-oxo-4-thia-1-azabicyclo[3.2.0]-hept-2-ene-2-carboxylate.

A 1 molar solution of sodium hydroxide in water was added to a solution of 360mg of 5(R),3-(3-aminosulphonyl-propylthio)-6(S)-[1(R)-hydroxyethyl]-7-oxo-4-thia-1-azabicyclo[3.2.0]hept-2-ene-2-carboxylic acid in 10 ml of acetonitrile and 10ml of water until the pH reached 6.3. The solution was frozen and lyophilised to leave the title compound as a pale yellow solid (374mg).

δ ($D_2O$) 5.70 (1H,d,J=1.4Hz); 4.32-4.18 (1H,m); 3.93 (1H,dd,J=6.0Hz and 1.4Hz); 3.48-3.31 (2H,m); 3.27-2.91 (2H,m); 2.32-2.14 (2H,m); 1.32 (3H,d,J=6.4Hz).

Example 7

1'-Acetoxyethyl 5(R),3-(3-aminosulphonyl-propylthio)-6(S)-[1(R)-hydroxyethyl]-7-oxo-4-thia-1-azabicyclo-[3.2.0]hept-2-ene-2-carboxylate.

A mixture of 250mg of potassium 5(R),3-(3-amino sulphonyl-propylthio)-6(S)-[1(R)-hydroxyethyl]-7-oxo-4-thia-1-azabicyclo[3.2.0]hept-2-ene-2-carboxylate, 184mg of sodium iodide and 226mg of 1-acetoxy-1-chloroethane in 0.5ml of dry dimethylformamide were stirred at room temperature for 24 hours. Water was added and the mixture extracted several times with

ethyl acetate. The combined organic extracts were washed with sodium bicarbonate solution and water and dried over anhydrous magnesium sulphate. The filtered solution was evaporated *in vacuo* and the residue chromatographed on silica using hexane/ethyl acetate mixtures as eluant to give 61mg of the title compound.

δ ($CDCl_3$) 6.97,6.94 (1H,2xq,J=5.4Hz); 5.66,5.67 (1H,2xd,J=1.5Hz); 4.99 (2H,br.s); 4.34-4.16 (1H,m); 3.75 (1H,dd,J=6.3 and 1.5Hz); 3.39-2.97 (4H,m); 2.40-2.25 (2H,m); 2.11,2.09 (3H,2xs); 1.66,1.64 (3H,2xd,J=5.4Hz); 1.38,1.36 (3H,2xd,J=6.3Hz).

Example 8

4-Nitrobenzyl 5(R),6(S)-[1(R)-acetoxyethyl]-3-(3-aminosulphonyl-propylthio)-7-oxo-4-thia-1-azabicyclo-[3.2.0]hept-2-ene-2-carboxylate.

234µl of acetic anhydride was added to a solution of 250mg of 4-nitrobenzyl 5(R),3-(3-aminosulphonyl-propylthio)-6(S)-[1(R)-hydroxyethyl]-7-oxo-4-thia-1-azabicyclo[3.2.0]hept-2-ene-2-carboxylate in 15ml of tetrahydrofuran. To this mixture was added 6mg of 4-dimethylaminopyridine and the solution stirred at room temperature until the reaction was complete as judged by t.l.c. Solvents were evaporated *in vacuo* and the residue taken up in ethyl acetate and the organic solution washed with water and brine and dried over anhydrous magnesium sulphate. Evaporation of the filtered solution and chromatography of the residue on silica gel using hexane/ethyl acetate mixtures as eluant gave 244mg of the title compound as a pale yellow solid.

$\gamma_{max}$ (Nujol mull) 1787,1737,1690 $cm^{-1}$

δ($CDCl_3$) 8.22,7.61 (4H,AA'BB',J=8.7Hz); 5.65 (1H,d,J=1.5Hz); 5.45,5.24 (2H,ABq,J=13.7Hz); 5.33-5.18

(1H,m); 4.69 (2H,br.s); 3.88 (1H,dd,J=7.5Hz and 1.5Hz); 3.32-3.21 (2H,m); 3.20-3.01 (2H,m); 2.38-2.23 (2H,m); 2.07 (3H,s); 1.42 (3H,d,J=6.5Hz).

Example 9

Potassium 5(R),6(S)-[1(R)-acetoxyethyl]-3-(3-aminosulphonyl-propylthio)-7-oxo-4-thia-1-azabicyclo[3.2.0]hept-2-ene-2-carboxylate.

A mixture of 170mg of the 4-nitrobenzyl carboxylate defined in Example 8 in 10ml of ethyl acetate, 31.2mg of potassium bicarbonate in 10ml of water and 170mg of 10% palladium on charcoal catalyst was hydrogenated at about 345 kPa (about 50 p.s.i) in a Parr apparatus for 75 minutes. The mixture was filtered through "Hyflo" and the aqueous layer separated, washed with ethyl acetate and lyophilised to give 74mg of the title compound.

$\gamma_{max}$ (KBr disc) 1775, 1738 $cm^{-1}$

$\delta(D_2O)$ 5.69 (1H,d,J=1.5Hz); 5.26-5.12 (1H,m); 4.07 (1H,dd,J=5.0Hz and 1.5Hz); 3.37-3.24 (2H,m); 3.19-3.00 (1H,m); 2.98-2.81 (1H,m); 2.25-2.07 (2H,m); 2.03 (3H,s); 1.28 (3H,d,J=6.3Hz).

Example 10

N-Methyl-3-chloropropanesulphonamide

Gaseous methylamine was passed into a stirred solution of 1.0g of 3-chloropropanesulphonyl chloride in dry diethyl ether (10ml) cooled to -10°C. The temperature was maintained at below 5°C during the addition and when the reaction was complete, as judged by t.l.c., additional ether was added and the mixture washed with water. The organic layer was separated, dried over anhydrous magnesium sulphate and evaporated

*in vacuo* to afford 0.62g of the title compound.

δ $(CDCl_3)$ 4.93 (1H,br); 3.68 (2H,t,J=6.5Hz); 3.18 (2H,dd,J=8.0Hz and 6.0Hz); 2.79 (3H,d,J=4.5Hz); 2.52-1.97 (2H,m).

Example 11

N-Methyl-3-iodopropanesulphonamide

Sodium iodide (1.09g) and N-methyl-3-chloropropanesulphonamide (0.62g) were dissolved in 5ml of dry acetone and the mixture refluxed for 2.5 hours. After cooling, the solution was partitioned between ethyl acetate and water. The aqueous layer was separated and extracted with a further portion of ethyl acetate. The combined organic layers were washed with an aqueous solution of sodium metabisulphite, water and finally dried over anhydrous magnesium sulphate. Evaporation of the filtered solution gave 0.70g of the title compound.

δ $(CDCl_3)$ 4.13 (1H,br); 3.31 (2H,t,J=6.6Hz); 3.24-3.10 (2H,m); 2.84 (3H,d,J=5.5Hz); 2.39-2.20 (2H,m).

Example 12

4-Nitrobenzyl 5(R),6(S)-[1(R)-hydroxyethyl]-3-(3-methylaminosulphonyl-propylthio)-7-oxo-4-thia-1-azabicyclo[3.2.0]hept-2-ene-2-carboxylate.

To a stirred solution of 0.15g of 4-nitrobenzyl 5(R),6(S)-[1(R)-hydroxyethyl]-7-oxo-4-thia-3-thioxo-1-azabicyclo[3.2.0]heptane-2-carboxylate in 1ml of dry tetrahydrofuran was added 0.103g of N-methyl-3-iodopropanesulphonamide in a little tetrahydrofuran followed by 0.15ml of diisopropylethylamine. The reaction mixture was stirred at room temperature for approximately 2 hours and the solvents evaporated.

Chromatography of the residue on deactivated silica gel using hexane/ethyl acetate mixtures as eluant gave the product as a white solid (0.133g).

$\delta$ ($d_6$-acetone) 8.23,7.72 (4H,AA'BB',J=8.9Hz); 6.12 (1H,br.m); 5.81 (1H,d,J=1.4Hz); 5.50,5.28 (2H,ABq,J=14.2Hz); 4.25-4.10 (1H,m); 3.85 (1H,dd,J=6.3Hz and 1.4Hz); 3.34-3.05 (4H,m); 2.71 (3H,d,J=5.1Hz); 2.25-2.13 (2H,m); 1.28 (3H,d,J=6.3Hz).

Example 13

Potassium 5(R),6(S)-[1(R)-hydroxyethyl]-3-(3-methylaminosulphonyl-propylthio)-7-oxo-4-thia-1-azabicylco[3.2.0]hept-2-ene-2-carboxylate.

A mixture of 130mg of the 4-nitrobenzyl carboxylate defined in Example 12 in 10ml of ethyl acetate, 25.1mg of potassium bicarbonate in 10ml of water and 260mg of 10% palladium on charcoal catalyst was hydrogenated at about 345 kPa (about 50 p.s.i.) in a Parr apparatus for 90 minutes. The mixture was filtered through "Hyflo" and the aqueous layer separated, washed with ethyl acetate and lyophilised to give 71mg of the title compound.

$\gamma_{max}$ (KBr disc) 1769 $cm^{-1}$

$\delta$ ($D_2O$) 5.68 (1H,d,J=1.4Hz); 4.32-4.16 (1H,m); 3.91 (1H,dd,J=6.1Hz and 1.4Hz); 3.45-3.28 (2H,m); 3.25-2.89 (2H,m); 2.72 (3H,s); 2.24-2.05 (2H,m); 1.29 (3H,d,J=6.3Hz).

Example 14

N,N-Dimethyl-3-chloropropanesulphonamide

Gaseous dimethylamine was bubbled into a stirred solution of 1.0g of 3-chloropropanesulphonyl chloride

in 10ml of dry ether . The solution was kept below $-5^{o}C$ by cooling until the reaction was complete. The mixture was allowed to reach room temperature, additional ether added and the organic phase washed with water and dried over anhydrous magnesium sulphate. Evaporation of the filtered solution afforded 1.02g of the title compound.

δ ($CDCl_3$) 3.68 (2H,t,J=6.0Hz); 3.06 (2H,dd,J=7.5Hz and 6.0Hz); 2.86 (6H,s); 2.50-1.94 (2H,m).

Example 15

N,N-Dimethyl-3-iodopropanesulphonamide

A mixture of 1.02g of N,N-dimethyl-3-chloropropanesulphonamide and 1.65g of sodium iodide were dissolved in 10ml of dry acetone and the mixture heated to reflux for 2 hours. After cooling to room temperature the mixture was partitioned between ethyl acetate and water. The aqueous layer was separated and extracted with a further portion of ethyl acetate and the combined organic extracts washed with an aqueous solution of sodium metabisulphite, water and then dried over anhydrous magnesium sulphate. The filtered solution was evaporated *in vacuo* to leave 1.29g of the title compound as a white solid.

δ($CDCl_3$) 3.33 (2H,t,J=6.0Hz); 3.18-2.69 (2H,m); 2.89 (6H,s); 2.57-2.01 (2H,m).

Example 16

4-Nitrobenzyl -5(R),3-(3-dimethylaminosulphonylpropylthio)-6(S)-[1(R)-hydroxyethyl]-7-oxo-4-thia-1-azabicyclo[3.2.0]hept-2-ene-2-carboxylate.

To a stirred solution of 0.15g of 4-nitrobenzyl

5(R),6(S)-[1(R)-hydroxyethyl]-7-oxo-4-thia-3-thioxo-1-azabicyclo[3.2.0]heptane-2-carboxylate in 1ml of dry tetrahydrofuran was added 0.109g of N,N-dimethyl-3-iodopropanesulphonamide in a little tetrahydrofuran followed by 0.15ml of diisopropylethylamine. After stirring at room temperature for 2 hours, t.l.c. analysis indicated the reaction was complete. The solvents were evaporated and the residue chromatographed on deactivated silica gel using hexane/ethyl acetate/dichloromethane mixtures as eluant to give 0.13g of the title compound as a white solid.

$\delta$ ($d_6$-acetone) 8.23,7.76 (4H,AA'BB',J=8.9Hz); 5.81 (1H,d,J=1.6Hz); 5.50,5.27 (2H,ABq,J=14.1Hz); 4.11-4.27 (1H,m); 3.86 (1H,dd,J,6.5Hz and 1.6Hz); 3.33-2.96 (4H,m); 2.84 (6H,s); 2.27-2.14 (2H,m); 1.28 (3H,d,J=6.3Hz).

Example 17

Potassium ~~5~~(R),3-(3-dimethylaminosulphonyl-propylthio)-6(S)-[1(R)-hydroxyethyl]-7-oxo-4-thia-1-azabicyclo-[3.2.0]hept-2-ene-2-carboxylate.

A mixture of 130mg of the 4-nitrobenzyl carboxylate defined in Example 16 in 10ml of ethyl acetate, 24.5mg of potassium bicarbonate in 10ml of water and 260mg of 10% palladium on charcoal catalyst was hydrogenated at about 345 kPa (about 50 p.s.i.) in a Parr apparatus for 100 minutes. The mixture was filtered through "Hyflo" and the aqueous layer separated, washed with ethyl acetate and lyophilised to give 74mg of the title compound.

$\gamma_{max}$ (KBr disc) 1768 $cm^{-1}$

$\delta$ ($D_2O$) 5.68 (1H,d,J=1.3Hz); 4.32-4.20 (1H,m); 3.91 (1H,dd,J=6.0Hz and 1.3Hz); 3.43-3.28 (2H,m); 3.26-2.84 (2H,m); 2.87 (6H,s); 2.28-2.11 (2H,m);

Example 18

N-(2-(4-Nitrobenzyloxycarbonylamino)ethyl)-3-chloropropanesulphonamide

A solution of 46mg of sodium hydroxide in 4ml of water was added to a mixture of 102mg of 3-chloropropanesulphonyl chloride and 159mg of N-(4-nitrobenzyloxycarbonyl)ethylene diamine hydrochloride in 10ml of diethyl ether. The mixture was stirred vigorously until the organic layer became clear. Water was added and the mixture extracted with ethyl acetate. The organic extracts were dried over anhydrous magnesium sulphate, filtered and evaporated *in vacuo* to give the title compound (121mg).

δ ($CDCl_3$) 8.22,7.51 (4H,AA'BB',J=8.5Hz); 5.21 (2H,s); 5.28,4.79 (2H,2 x br.s); 3.67 (2H,t,J=6.1Hz); 3.48-3.17 (6H,m); 2.36-2.21 (2H,m).

Example 19

N-(2-(4-Nitrobenzyloxycarbonylamino)ethyl)-3-iodopropanesulphonamide.

A mixture of 79mg of sodium iodide and 101mg of the 3-chloropropanesulphonamide defined in Example 18 in 5ml of dry acetone were heated at reflux until t.l.c. analysis indicated the reaction was complete. The solvent was evaporated and the residue partitioned between ethyl acetate and water. The organic layer was separated and the aqueous layer extracted with a further portion of ethyl acetate. The combined organic layers wre washed with an aqueous solution of sodium metabisulphite, water and finally dried over anhydrous magnesium sulphate. The filtered solution was evaporated to leave 114mg of the title compound.

δ ($d_6$-acetone) 8.23,7.64 (4H,AA'BB',J=8.6Hz); 6.67,6.28 (2H,2 x br.s); 5.23 (2H,s); 3.47-3.13 (8H,m); 2.35-2.16 (2H,m).

Example 20

4-Nitrobenzyl 5(R),6(S)-[1(R)-hydroxyethyl]-3-[3-(2-(4-nitrobenzyloxycarbonylamino)ethylaminosulphonyl)-propylthio]-7-oxo-4-thia-1-azabicyclo[3.2.0]hept-2-ene-2-carboxylate.

To a stirred solution of 90.3mg of 4-nitrobenzyl 5(R),6(S)-[1(R)-hydroxyethyl]-7-oxo-4-thia-3-thioxo-1-azabicyclo[3.2.0]heptane-2-carboxylate in 2ml of dry tetrahydrofuran was added a solution of 111.4mg of the 3-iodopropylsulphonamide defined in Example 19 in 2ml of tetrahydrofuran followed by 45µl of diisopropylethylamine. The reaction mixture was stirred at room temperature for 2 hours when solvents were evaporated and the residue partitioned between ethyl acetate and water. The separated organic layer was washed with water, dried over anhydrous magnesium sulphate, filtered and evaporated *in vacuo*. Chromatography of the residue on silica gel using ethyl acetate/hexane mixtures as eluant gave 106.6mg of the title compound.

δ ($d_6$-acetone) 8.23,7.76 (4H,AA'BB',J=8.9Hz); 8.22,7.63 (4H,AA'BB',J=8.7Hz); 6.72,6.30 (2H,2 x br.s); 5.82 (1H,d,J=1.5Hz); 5.50,5.26 (2H,ABq,J=14.2Hz); 5.23 (2H,s); 4.28-4.10 (1H,m); 3.86 (1H,dd,J=6.3 and 1.5Hz); 3.45-3.08 (8H,m); 2.29-2.14 (2H,m); 1.29 (3H,d,J=6.3Hz).

0211236

Example 21

Potassium 5(R),3-(3-(2-aminoethylaminosulphonyl)-propylthio)-6(S)-[1(R)-hydroxyethyl]-7-oxo-4-thia-1-azabicyclo[3.2.0]hept-2-ene-2-carboxylate.

A mixture of 103.1mg of the 4-nitrobenzyl carboxylate defined in Example 20 in 8ml of ethyl acetate, 14.2mg of potassium bicarbonate in 8ml of water and 206mg of 10% palladium on charcoal catalyst was hydrogenated at about 345 kPa (about 50 p.s.i.) in a Parr apparatus for 60 minutes. The mixture was filtered through "Hyflo" and the aqueous layer separated, washed with ethyl acetate and lyophilised to give 44.8mg of the title compound.

δ ($D_2O$) 5.67 (1H,d,J=1.5Hz); 4.34-4.14 (1H,m); 3.91 (1H,dd,J=5.9Hz and 1.5Hz); 3.42-2.86 (8H,m); 2.22-2.04 (2H,m); 1.29 (3H,d,J=6.4Hz).

Example 22

4-Nitrobenzyl 5(R),3-ethylthio-6(S)-[1(R)-hydroxyethyl]--7-oxo-4-thia-1-azabicyclo[3.2.0]hept-2-ene-2-carboxylate.

To a solution of 260mg of 4-nitrobenzyl 5(R),6(S)-[1(R)-hydroxyethyl]-7-oxo-4-thia-3-thioxo-1-azabicyclo-[3.2.0]heptane-2-carboxylate and 0.11ml of iodoethane in 10ml of dry tetrahydrofuran was added 0.13ml of diisopropylethylamine. The mixture was stirred at room temperature for 14 hours, evaporated *in vacuo* and the residue chromatographed on silica gel using diethyl ether/hexane mixtures as eluant to give 210mg of the title compound.

δ ($CDCl_3$) 8.22,7.62 (4H,AA'BB',J=8.8Hz) 5.67 (1H,d,J=1.5Hz); 5.47,5.22 (2H,ABq,J=13.8Hz); 4.33-4.21 (1H,m); 3.76 (1H,dd,J=6.6Hz and 1.5Hz);

3.14-2.87 (2H,m); 2.06 (1H br.s); 1.39 (3H,t,J=7.4Hz); 1.38 (3H,d,J=6.4Hz).

Example 23

4-Nitrobenzyl 5(R),3-ethylsulphinyl-6(S)-[1(R)-hydroxyethyl]-7-oxo-4-thia-1-azabicyclo[3.2.0]hept-2-ene-2-carboxylate.

A solution of 362mg of *meta*-chloroperbenzoic acid (85% pure) in 10ml of dichloromethane was added to a solution of 732mg of 4-nitrobenzyl 5(R),3-ethylthio-6(S)-[1(R)-hydroxyethyl]-7-oxo-4-thia-1-azabicyclo-[3.2.0]hept-2-ene-2-carboxylate in 20ml of dichloromethane, the mixture being kept at -40°C by external cooling. The mixture was stirred at -40°C for 10 minutes and then allowed to reach room temperature and stirred for a further 2 hours. The organic phase was washed succesively with a solution of potassium metabisulphite, a solution of sodium bicarbonate and water and dried over anhydrous magnesium sulphate. Evaporation of the filtered solution gave a residue that was chromatographed on silica gel using ethyl acetate/hexane mixtures as eluant to give 345 mg of the title compound as a mixture of R and S isomers at the oxidised sulphur atom.

δ($CDCl_3$) 8.24,7.62 and 8.24,7.60 (4H,2xAA'BB', J=8.8Hz); 5.87,5.75 (1H,2xd,J=1.8Hz); 5.45,5.24 (2H,ABq, J=13.6Hz); 4.36-4.19 (1H,m); 3.98-3.91 (1H,m); 3.20-3.02 (2H,m); 1.43 (3H,t,J=7.5Hz); 1.39 (3H,d, J=6.5Hz).

Example 24

3-Aminosulphonylpropyl carbamimidothioate hydrochloride

A solution of 2.02g of thiourea and 4.18g of 3-chloropropanesulphonamide in 42ml of water was heated under reflux for 5 hours. The water was evaporated at 80°C *in vacuo* to afford the title compound as a white solid.

Example 25

3-Mercaptopropanesulphonamide

A solution of 1.08g of sodium hydroxide in 15ml of water was added to a solution of 4.52g of the isothiouronium salt defined in Example 24 in 15ml of ethanol and the mixture heated to reflux for 4 hours. The cooled solution was acidified with hydrochloric acid and extracted with portions of ethyl acetate. The combined organic layers were dried over anhydrous magnesium sulphate, filtered and evaporated *in vacuo*. The residue was chromatographed on silica gel using hexane/ethyl acetate mixtures as eluant to afford 1.65g of the title compound.

$\delta$ ($CDCl_3$) 4.67 (2H,br.s); 3.36-3.23 (2H,m); 2.79-2.64 (2H,m); 2.27-2.10 (2H,m); 1.42 (1H,t,J=8.3Hz).

Example 26

4-Nitrobenzyl 5(R),3-(3-aminosulphonyl-propylthio)-6(S)-[1(R)-hydroxyethyl]-7-oxo-4-thia-1-azabicyclo-[3.2.0]hept-2-ene-2-carboxylate.

A mixture of 83mg of the ethylsulphinyl compound defined in Example 23, 32mg of 3-mercaptopropanesulphonamide and 39µl of diisopropylethylamine in 4ml of acetonitrile was stirred at room temperature for 17 hours, when the

reaction appeared complete as judged by t.l.c.. Evaporation *in vacuo* of volatile materials followed by chromatography of the residue on silica gel using hexane/ethyl acetate mixtures as eluant gave 53mg of the title compound.

$\gamma_{max}$ (KBr disc) 1768 $cm^{-1}$

$\delta$($d_6$-acetone) 8.24,7.77 (4H,AA'BB',J=8.9Hz); 6.18 (2H,br.s); 5.81 (1H,d,J=1.5Hz); 5.51,5.28 (2H,ABq,J=14.1Hz); 4.30-4.11 (1H,m); 3.85 (1H,dd,J=6.3Hz and 1.5Hz); 3.34-3.07 (4H,m); 2.35-2.18 (2H,m); 1.29 (3H,d,J=6.3Hz).

This material was converted into the corresponding potassium carboxylate defined in Example 4 according to the procedure described in Example 4.

## Example 27

## 3(S)-[1(R)-Dimethyl-(2-methylprop-2-yl)silyloxyethyl]-4(R)-[(ethylthio)-carbonothioylthio]-azetidin-2-one.

460µl of ethanethiol was added to a solution of 150mg of sodium in 20ml of dry isopropanol cooled to 0°C. The cool solution was stirred at 0°C for 30 minutes, 375µl of carbon disulphide was added and the pale yellow mixture stirred for 15 minutes. Following this, a solution of 2.08g of 4(R)-acetoxy-3(S)-[1(R)-dimethyl-(2-methylprop-2-yl)silyloxyethyl]azetidin-2-one in 10ml of tetrahydrofuran was added to the mixture and the solution stirred at room temperature for 2 hours. The mixture was diluted with diethyl ether, water added and the organic layer separated. The aqueous layer was extracted with a further portion of ether and the combined organic layers washed with water and brine and dried over anhydrous magnesium sulphate. The filtered solution was evaporated *in vacuo* and the yellow residue chromatographed on silica gel using

0211236

hexane/diethyl ether mixtures as eluant to afford 1.87g of the title compound as a white solid.

$\nu_{max}$ (Nujol mull) 3610,3420 and 1775 $cm^{-1}$

δ ($CDCl_3$) 6.63 (1H,s); 5.67 (1H,d,J=2.5Hz); 4.37-4.21 (1H,m); 3.37 (2H,q,J=7.3Hz); 3.25-3.18 (1H,m); 1.37 (3H,t,J=7.3Hz); 1.22 (3H,d,J=6.3Hz); 0.88 (9H,s); 0.08 (6H,s).

Example 28

4-Nitrobenzyl 2-[3(S)-[1(R)-dimethyl-(2-methylprop-2-yl)-silyloxyethyl]-4(R)-[(ethylthio)-carbonothioylthio]-azetidin-2-on-1-yl]-2-oxoacetate.

A solution of 2.00g of the azetidinone defined in Example 27 in 30ml of dry dichloromethane containing 1.37g of suspended calcium carbonate was stirred and cooled to ca 4°C. To this solution was added 1.60g of 4-nitrobenzyl oxalyl chloride followed by a solution of 1.14ml of diisopropylethylamine in 9ml of dichloromethane, the latter being added slowly via a syringe pump over 2 hours. The yellow mixture was stirred for a further 30 minutes, filtered and the solids washed with a little dichloromethane. The combined filtrates were washed with ice cold water, dried over anhydrous sodium sulphate, filtered and evaporated *in vacuo* to afford a quantitative yield of the title compound as a pale yellow gum. The material was not purified but used directly in the next step.

δ ($CDCl_3$) 8.22,7.56 (4H,AA'BB',J=8.5Hz); 6.74 (1H,d,J=3.0Hz); 5.41 (2H,s); 4.54-4.20 (1H,m); 4.40 (1H,dd,J=6.5Hz and 3.0Hz); 3.42 (2H,q,J=7Hz); 1.38 (3H,t,J=7Hz); 1.34 (3H,d,J=6.5Hz); 0.81 (9H,s); 0.06 (6H,s).

Example 29

4-Nitrobenzyl 5(R),6(S)-[1(R)-dimethyl-(2-methylprop-2-yl)silyloxyethyl]-3-ethylthio-7-oxo-4-thia-1-azabicyclo[3.2.0]hept-2-ene-2-carboxylate.

A solution of 1.57g of the acylated azetidinone described in Example 28 in 20ml of dry dichloromethane was heated to reflux temperature and treated slowly dropwise with 646µl of trimethyl phosphite. The mixture was heated at reflux in the absence of moisture until t.l.c. analysis indicated the reaction was complete. The mixture was evaporated and the residue placed under high vacuum. Chromatography of the residue on silica gel using hexane/diethyl ether mixtures as eluant gave 703mg of the title penem.

δ($CDCl_3$) 8.21,7.63 (4H,AA'BB',J=8.6Hz); 5.65 (1H,d,J=1.4Hz); 5.43,5.22 (2H,ABq,J=13.8Hz); 4.35-4.16 (1H,m); 3.72 (1H,dd,J=4.3Hz and 1.4Hz); 3.14-2.90 (2H,m); 1.39 (3H,t,J=7.5Hz); 1.26 (3H,d,J=6.3Hz); 0.84 (9H,s); 0.07 (3H,s); 0.04 (3H,s).

Example 30

4-Nitrobenzyl 5(R),6(S)-[1(R)-dimethyl-(2-methylprop-2-yl)silyloxyethyl]-3-ethylsulphinyl-7-oxo-4-thia-1-azabicyclo[3.2.0]hept-2-ene-2-carboxylate.

Solid *m*-chloroperbenzoic acid (336mg) was added to a stirred solution of 692mg of 4-nitrobenzyl 5(R),6(S)-[1(R)-dimethyl-(2-methylprop-2-yl)-silyloxyethyl]-3-ethylthio-7-oxo-4-thia-1-azabicyclo-[3.2.0]hept-2-ene-2-carboxylate in 20ml of dichloromethane kept at -40°C by immersion in a cooling bath. After allowing the mixture to reach room temperature stirring was continued for 2 hours. Ethyl acetate was added and the organic layer washed

successively with solutions of aqueous potassium metabisulphite, sodium bicarbonate and finally water. Evaporation of the dried (anhydrous magnesium sulphate) solution afforded a brown residue which was chromatographed on silica gel using hexane/ethyl acetate mixtures as eluant to give 295mg of title compound as a mixture of R and S isomers at the oxidised sulphur atom.

δ ($CDCl_3$) 8.22,7.62 and 8.22,7.59 (4H,2xAA'BB', J=8.8Hz); 5.84,5.72 (1H,2xd,J=1.8Hz); 5.56-5.22 (2H,m); 4.35-4.21 (1H,m); 3.96-3.85 (1H,m); 3.19-3.01 (2H,m); 1.44,1.43 (3H,2xt,J=7.4Hz); 1.25 (3H,d,J=6.2Hz); 0.84,0.83 (9H,2xs); 0.08,0.07,0.04, 0.01 (6H,4xs).

Example 31

N,N-Dimethyl 3-mercaptopropanesulphonamide

A mixture of 1.01g of N,N-dimethyl 3-iodopropanesulphonamide and 278mg of thiourea were heated at reflux in 10ml of water for 1 hour. The solution was cooled slightly and 219mg of sodium hydroxide added followed by 10ml of ethanol. The mixture was again heated to reflux until the reaction was complete as evidenced by t.l.c. After cooling the mixture was acidified with hydrochloric acid and extracted with portions of ethyl acetate. The combined extracts were dried over anhydrous magnesium sulphate, filtered and evaporated *in vacuo*. Chromatography of the residue on silica gel using ethyl acetate/hexane mixtures as eluant gave 337mg of the title compound.

δ ($CDCl_3$) 3.24-3.01 (2H,m); 2.89 (6H,s); 2.77-2.58 (2H,m); 2.21-2.03 (2H,m); 1.40 (1H,t,J=8.2Hz)

Example 32

4(R)-[(3-Dimethylaminosulphonyl-propylthio)-carbonothioylthio]-3(S)-[1(R)-dimethyl-(2-methylprop-2-yl)silyloxyethyl]-azetidin-2-one.

A solution of 337mg of N,N-dimethyl 3-mercaptopropanesulphonamide in 2ml of isopropanol was added to a stirred solution of 44mg of sodium in 8ml of dry isopropanol. The mixture was stirred at room temperature for 40 minutes and 111µl of carbon disulphide added and stirring continued for another 30 minutes. Following this, a solution of 614mg of 4(R)-acetoxy-3(S)-[1(R)-dimethyl-(2-methylprop-2-yl)silyloxyethyl]azetidin-2-one in 4ml of tetrahydrofuran was added and the solution cooled to 0°C. Stirring was continued until t.l.c. indicated the reaction to be complete when the mixture was diluted with diethyl ether and water. The separated water layer was extracted with a further portion of ether and the combined organic layers washed with water and brine and dried over anhydrous magnesium sulphate. Evaporation of the filtered solution gave a residue which on chromatography on silica gel using hexane/ethyl acetate mixtures as eluant gave 456mg of the title compound.

δ ($CDCl_3$) 6.55 (1H,br.s); 5.68 (1H,d,J=2.5Hz); 4.38-4.20 (1H,m); 3.54 (2H,t,J=7.2Hz); 3.29-3.18 (1H,m); 3.01 (2H,t,J=7.1Hz); 2.89 (6H,s); 2.34-2.15 (2H,m); 1.22 (3H,d,J=6.3Hz); 0.88 (9H,s); 0.08 (6H,s).

3R

Example 33

4-Nitrobenzyl 2-[4(R)-[(3-dimethylaminosulphonyl-propylthio)-carbonothioylthio]-3(S)-[1(R)-dimethyl-(2-methylprop-2-yl)silyloxyethyl]-azetidin-2-on-1-yl]-2-oxoacetate.

A solution of 150mg of the azetidinone defined in Example 32 and 131mg of 4-nitrobenzyl oxalyl chloride in 3ml of dry dichloromethane containing 86mg of calcium carbonate was stirred and cooled to 5°C in the absence of atmospheric moisture. A solution of 94µl of diisopropylethylamine in 1ml of dichloromethane was added to the mixture and the solution stirred at 5°C for 90 minutes when the reaction was complete. The filtered solution was washed with water, dried over anhydrous sodium sulphate, filtered and evaporated to afford a quantitative yield of the title compound. This material was used without further purification in the next step.

δ($CDCl_3$) 8.24,7.56 (4H,AA'BB',J=8.8Hz); 6.74 (1H,d,J=2.5Hz); 5.39 (2H,br.s); 4.48-4.24 (1H,m); 4.39 (1H,dd,J=6.4Hz and 2.5Hz); 3.68-3.50 (2H,m); 3.02 (2H,t,J=7.2Hz); 2.88 (6H,s); 2.40-2.15 (2H,m); 1.24 (3H,d,J=6.4Hz); 0.83 (9H,s); 0.09 (6H,s).

Example 34

4-Nitrobenzyl 5(R),3-(3-dimethylaminosulphonyl-propylthio)-6(S)-[1(R)-dimethyl-(2-methylprop-2-yl)-silyloxyethyl]-7-oxo-4-thia-1-azabicyclo[3.2.0]hept-2-ene-2-carboxylate.

A solution of 220mg of the acylated azetidinone defined in Example 33 in 5ml of dry chloroform was gently heated to reflux and 111µl of triethyl phosphite added and the yellow mixture continued to be heated and

stirred until the reaction was judged complete by t.l.c. Volatile solvents were evaporated *in vacuo* and the residue chromatographed on silica gel using hexane/ ethyl acetate mixtures as eluant to give 77mg of the title compound.

δ ($CDCl_3$) 8.21,7.62 (4H,AA'BB',J=8.7Hz); 5.67 (1H,d,J=1.4Hz); 5.42,5.21 (2H,ABq,J=13.7Hz); 4.38-4.22 (1H,m); 3.73 (1H,dd,J=4.1Hz and 1.4Hz); 3.29-2.96 (4H,m); 2.88 (6H,s); 2.35-2.18 (2H,m); 1.25 (3H,d,J=6.3Hz); 0.83 (9H,s); 0.07,0.04 (6H,2xs).

Example 35

4-Nitrobenzyl 5(R),3-(3-dimethylaminosulphonyl-propylthio)-6(S)-[1(R)-dimethyl-(2-methylprop-2-yl)-silyloxyethyl]-7-oxo-4-thia-1-azabicyclo[3.2.0]hept-2-ene-2-carboxylate.

A mixture of 101mg of the ethylsulphinyl penem defined in Example 30, 48mg of N,N-dimethyl 3-mercaptopropanesulphonamide and 42µl of diisopropylethylamine in 5ml of acetonitrile was stirred at room temperature until t.l.c. indicated reaction was complete. Volatile solvents were evaporated *in vacuo* and the residue chromatographed on silica gel using hexane/ethyl acetate mixtures as eluant to give 59mg of the title compound, indentical to that prepared in Example 34.

Example 36

4-Nitrobenzyl 5(R),3-(3-dimethylaminosulphonyl-propylthio)-6(S)-[1(R)-hydroxyethyl]-7-oxo-4-thia-1-azabicyclo[3.2.0]hept-2-ene-2-carboxylate.

A solution of 3 molar equivalents of tetra-*n*-butylammonium fluoride in tetrahydrofuran was added to

a stirred mixture of 50mg of the silyloxyethyl penem defined in Examples 34 and 35, and 44μl of acetic acid in 5ml of tetrahydrofuran. The mixture was stirred at room temperature until reaction was complete as judged by t.l.c., the bulk of the solvents evaporated <u>in vacuo</u> and the residue chromatographed on silica gel using hexane/ethyl acetate mixtures as eluant to give 19.4mg of the title compound identical in all properties investigated to that produced in Example 16.

Claims

1. A compound of formula I

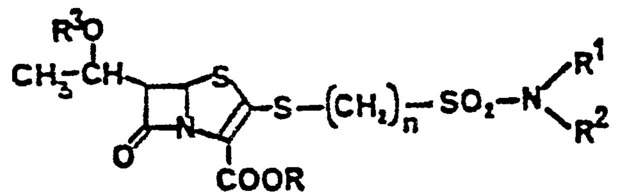

(I)

in which

n represents an integer of from 3 to 6;

R represents a hydrogen atom or a carboxylic acid esterifying group;

$R^1$ and $R^2$, which may be the same or different, each repesents an amino protecting group, a hydrogen atom, a straight or branched chain alkyl group having from 1 to 4 carbon atoms, or a cycloalkyl group having from 3 to 6 carbon atoms,

an alkyl or cycloalkyl group being unsubstituted or substituted by an $NH_2$ group which may be protected if desired or required; or

$R^1$ and $R^2$ form an alkylene group having from 3 to 5 carbon atoms; and

$R^3$ represents a hydrogen atom or a hydroxy protecting group;

and salts thereof.

2. A compound as claimed in claim 1, wherein $R^1$ and $R^2$ both represent hydrogen atoms.

3. A compound as claimed in claim 1, wherein $R^1$ and $R^2$ both represent methyl groups, or one represents a hydrogen atom and the other represents a methyl group or a 2-aminoethyl group.

4. A compound as claimed in claim 1, wherein
(i) n represents the integer 3 and $R^1$ and $R^2$ both represent hydrogen atoms;
(ii) n represents the integer 3, $R^1$ represents a hydrogen atom and $R^2$ represents a methyl group;
(iii) n represents the integer 3, and $R^1$ and $R^2$ both represent methyl groups; or
(iv) n represents the integer 3, $R^1$ represents a hydrogen atom and $R^2$ represents a 2-aminoethyl group.

5. A compound of formula I as claimed in any one of claims 1 to 4, wherein a protected carboxy group -COOR is an esterified carboxy group that can be converted by hydrolysis, by photolysis, by oxidation, by reduction or by esterase action to give the free acid of formula I or a carboxylate, for example the group R is a phenyl group or a methyl group substituted by one or more unsubstituted or substituted phenyl groups; and wherein a phenyl group, either as R or as a substituent of a methyl group, is optionally subsituted by one or more substituents selected from methoxy and nitro groups and halogen atoms, for example, R represents a benzyl, nitrobenzyl, methoxybenzyl, dimethoxybenzyl, benzhydryl or trityl group; or R represents a phenoxyethyl or trichloroethyl group; or R represents a trialkysilyl or trialkylsilyalkyl group in which each alkyl moiety has from 1 to 4 carbon atoms; or R represents a phthalidyl group; or R represents a methyl or ethyl group optionally subsituted by an acyl or acyloxy group, by an alkoxycarbonyloxy group, by an aminoalkanoyloxy group or by an optionally subsituted amino group, for example, R represents an acyloxymethyl or acyloxyethyl group having from 2 to 12 carbon atoms in the acyl moiety, an aminoalkanoyloxymethyl group having from 2 to 12 carbon atoms in the alkanoyl moiety, a 1'-(alkoxycarbonyloxy)ethyl

group, or an optionally substituted 2-aminoethyl group, especially a glycyloxymethyl, L-valinyloxymethyl or L-leucyloxymethyl group, a 1'-(methoxycarbonyloxy)ethyl group, a 2-diethylaminoethyl or 2-(1-morpholino)-ethyl group, a pivaloyloxymethyl, ethoxycarbonyloxymethyl, acetylmethyl, acetoxymethyl, 1'-(acetoxy)ethyl, 1'-(acetyl)ethyl or 1'-(ethoxycarbonyloxy)ethyl group; or R represents a 5-methyl-dioxalen-2-on-4-yl-methyl group.

6. A compound of formula I as claimed in any one of claims 1 to 5, wherein a hydroxy protecting group $R^3$ is a group that can be converted by hydrolysis, by photolysis, by reduction or by esterase enzyme action to give a compound of formula I having a free 8-hydroxy group, for example, $R^3$ is a carboxylic acid group of the formula $R^{20}CO-$ in which $R^{20}$ represents a hydrogen atom or a straight or branched chain alkyl group having from 1 to 6 carbon atoms, or represents a phenyl group or a phenoxyalkyl group in which the alkyl moiety is straight-chained or branched and has from 1 to 4 carbon atoms, for example, $R^{20}$ represents a methyl, ethyl or t-butyl group, or a phenoxymethyl group.

7. A compound of formula I as claimed in any one of claims 1 to 6, wherein R represents a hydrogen atom or a group that can be cleaved <u>in vivo</u> to give a free carboxy group or a carboxylate group, and $R^3$ represents a hydrogen atom or a group that can be cleaved <u>in vivo</u> to give a free hydroxy group.

8. A compound as claimed in any one of claims 1 to 7 having, independently or in any combination, 5R-stereochemisty, 6S-stereochemisty and 8R-stereochemistry, especially 5R,6S,8R-stereochemistry.

9. 5(R),3-(3-Dimethylaminosulphonyl-propylthio)-6(S)-[1(R)-hydroxyethyl]-7-oxo-4-thia-1-azabicyclo[3.2.0]hept-2-ene-2-carboxylic acid, or a salt thereof, or an ester thereof at the 2- and/or 8-position.

10. 5(R),6(S)-[1(R)-Hydroxyethyl]-3-(3-methylamino-sulphonyl propylthio)-7-oxo-4-thia-1-azabicyclo[3.2.0]hept-2-ene-2-carboxylic acid, or a salt thereof, or an ester thereof at the 2- and/or 8-position.

11. 5(R),3-(3-Aminosulphonyl-propylthio)-6(S)-[1(R)-hydroxyethyl]-7-oxo-4-thia-1-azabicyclo[3.2.0]hept-2-ene-2-carboxylic acid, or a salt thereof, or an ester thereof at the 2- and/or 8-position.

12. A process for the production of a compound of formula I or an ester or salt thereof as claimed in claim 1, which comprises

(i) reacting a compound of formula IIa or its tautomer of formula IIb or a mixture thereof

(IIa) 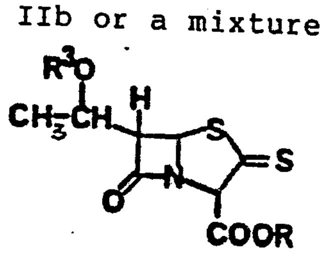

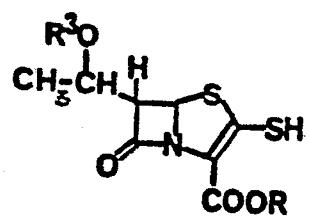 (IIb)

in which R is as defined in claim 1, and $R^3$ represents a hydrogen atom or a hydroxy protecting group, with a compound of formula III

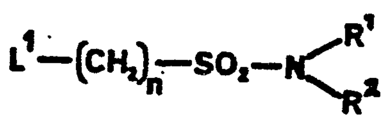 (III)

in which n, $R^1$ and $R^2$ are as defined in claim 1, and $L^1$ represents a leaving group, for example, a halogen atom, a modified hydroxy group, a triphenylphosphoniumoxy

group or an $-OCOCF_3$ group; or

(ii) treating a compound of formula IV

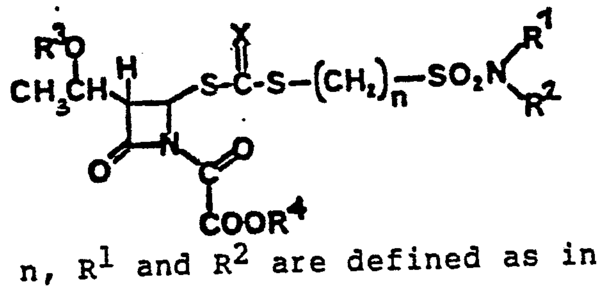

(IV)

in which n, $R^1$ and $R^2$ are defined as in claim 1, $R^4$ represents a carboxy protecting group, and X represents an oxygen atom or a sulphur atom, with a trivalent organophosphorus reagent to effect cyclisation to give a compound of formula I; or

(iii) reacting a compound of formula V

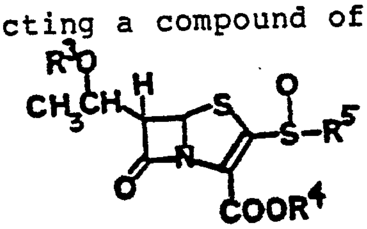

(V)

in which $R^3$ and $R^4$ are defined as above, and $R^5$ represents a straight or branched chain alkyl group having from 1 to 6 carbon atoms which may be substituted by one or more fluorine atoms, or represents an aryl group as hereinbefore defined, especially a phenyl group, with a compound of formula VI

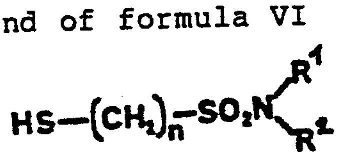

(VI)

in which n, $R^1$ and $R^2$ are defined as in claim 1, generally in the presence of a base, or with a reactive derivative of a compound of formula VI and, if desired, carrying out any one or more of the following steps in any desired order:

a) hydrolysing a 2-carboxylic ester of formula I to give the corresponding free acid of formula I,

b) reacting a free acid of formula I or a salt thereof

with an agent capable of forming a 2-carboxylic acid ester, for example, with an alcohol, a phenol or a reactive derivative thereof to give a 2-carboxylic acid ester of formula I,

c) carrying out an acid or base catalysed ester interchange on 2-carboxylic acid ester of formula I to give a different ester of formula I,

d) reacting a free acid of formula I with a base to give a salt at the carboxy group at position 2,

e) reacting a free acid of formula I or a 2-carboxylic acid ester of formula I having a basic group present with an acid to give an acid addition salt thereof,

f) hydrolysing the ester group from a 2-carboxylic acid ester of formula I in the presence of a salt-forming agent, for example, an alkali metal salt, to give a salt of a compound of formula I at the 2-carboxy group,

g) reacting a salt of a compound of formula I with an acid to give a free acid of formula I,

h) reacting a free acid of formula I or a salt thereof, or a 2-carboxylic acid ester of formula I, with an organic acid derivative to give a free acid of formula I or a 2-carboxylic acid ester of formula I having an esterified hydroxy group at the 8-position,

i) removing a carboxylic acid acyl group or a hydroxy protecting group from a modified 8-hydroxy group to give a compound of formula I having a free 8-hydroxy group, and

j) removing any amino protecting groups present.

13. A pharmaceutical preparation which comprises a compound of formula I as claimed in any one of claims 1 to 11, or a physiologically tolerable salt thereof, or a mixture of two or more such substances as active ingredient, in admixture or conjunction with a pharmaceutically suitable carrier.

14. A compound of formula I as claimed in claim 1 or a physiologically tolerable ester or salt thereof for use in the manufacture of a medicament for the treatment of bacterial infections.

15. A method of treating mammals, especially humams, to combat a bacterial infection, which comprises administering to the mammal a therapeutically effective amount of a compound of formula I as claimed in claim 1, or a physiologically tolerable ester or salt thereof.

16. A compound of formula IV

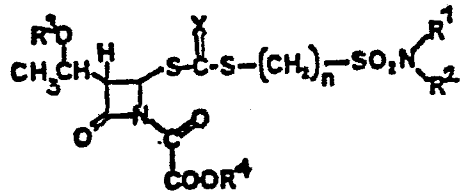

(IV)

in which n, $R^1$ and $R^2$ are as defined in claim 1, and X, $R^3$ and $R^4$ are as defined in claim 12.

17. A compound of formula XIII

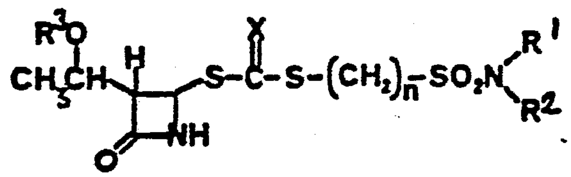

(XIII)

in which n, $R^1$ and $R^2$ are as defined in claim 1 and X is as defined in claim 12.

Patent Claims for Austria:

1. A process for the production of a compound of formula I

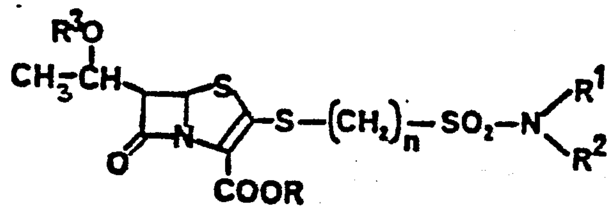
(I)

in which

n represents an integer of from 3 to 6;

R represents a hydrogen atom or a carboxylic acid esterifying group;

$R^1$ and $R^2$, which may be the same or different, each repesents an amino protecting group, a hydrogen atom, a straight or branched chain alkyl group having from 1 to 4 carbon atoms, or a cycloalkyl group having from 3 to 6 carbon atoms, an alkyl or cycloalkyl group being unsubstituted or substituted by an $NH_2$ group which may be protected if desired or required; or

$R^1$ and $R^2$ form an alkylene group having from 3 to 5 carbon atoms; and

$R^3$ represents a hydrogen atom or a hydroxy protecting group;

and salts thereof, which comprises

(i) reacting a compound of formula IIa or its tautomer of formula IIb or a mixture thereof

(IIa) 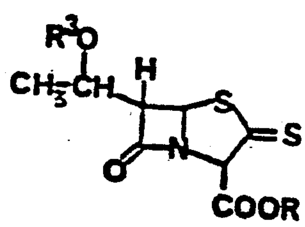 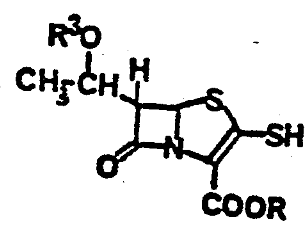 (IIb)

in which R is as defined above, and $R^3$ represents a hydrogen atom or a hydroxy protecting group, with a compound of formula III

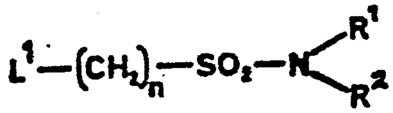

(III)

in which n, $R^1$ and $R^2$ are as defined above, and $L^1$ represents a leaving group, or

(ii) treating a compound of formula IV

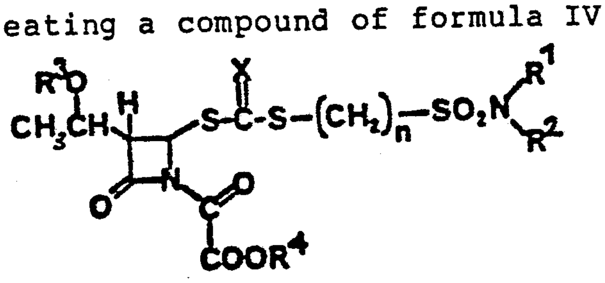

(IV)

in which n, $R^1$ and $R^2$ are defined above, $R^4$ represents a carboxy protecting group, and X represents an oxygen atom or a sulphur atom, with a trivalent organophosphorus reagent to effect cyclisation to give a compound of formula I; or

(iii) reacting a compound of formula V

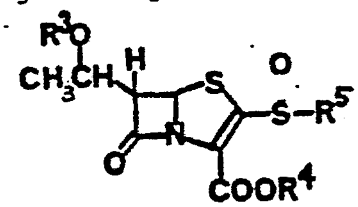

(V)

in which $R^3$ and $R^4$ are defined as above, and $R^5$ represents a straight or branched chain alkyl group having from 1 to 6 carbon atoms which may be substituted by one or more fluorine atoms, or represents an aryl group as hereinbefore defined, especially a phenyl group, with a compound of formula VI

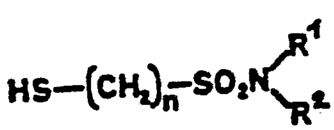

(VI)

in which n, $R^1$ and $R^2$ are defined as above, generally in the presence of a base, or with a reactive derivative of a compound of formula VI and, if desired, carrying out any one or more of the following steps in any desired order:

a) hydrolysing a 2-carboxylic ester of formula I to give the corresponding free acid of formula I,

b) reacting a free acid of formula I or a salt thereof with an agent capable of forming a 2-carboxylic acid ester, for example, with an alcohol, a phenol or a reactive derivative thereof to give a 2-carboxylic acid ester of formula I,

c) carrying out an acid or base catalysed ester interchange on 2-carboxylic acid ester of formula I to give a different ester of formula I,

d) reacting a free acid of formula I with a base to give a salt at the carboxy group at position 2,

e) reacting a free acid of formula I or a 2-carboxylic acid ester of formula I having a basic group present with an acid to give an acid addition salt thereof,

f) hydrolysing the ester group from a 2-carboxylic acid ester of formula I in the presence of a salt-forming agent, for example, an alkali metal salt, to give a salt of a compound of formula I at the 2-carboxy group,

g) reacting a salt of a compound of formula I with an acid to give a free acid of formula I,

h) reacting a free acid of formula I or a salt thereof, or a 2-carboxylic acid ester of formula I, with an organic acid derivative to give a free acid of formula I or a 2-carboxylic acid ester of formula I having an esterified hydroxy group at the 8-position,

i) removing a carboxylic acid acyl group or a hydroxy protecting group from a modified 8-hydroxy group to give a compound of formula I having a free 8-hydroxy group,

and

j) removing any amino protecting groups present.

2. A process as claimed in claim 1, wherein $L^1$ in formula III represents a halogen atom, preferably a bromine or iodine atom; a modified hydroxy group, especially a sulphonate ester group, for example, a sulphonyloxy group of the formula $-OSO_2R^6$ in which $R^6$ represents a straight or branched chain alkyl group having from 1 to 4 carbon atoms, which may be substituted by one or more fluorine atoms, for example, a $-CF_3$ or $-C_4F_9$ group, or $R^6$ represents a phenyl group that is unsubstituted or is substituted by a p-nitro, p-bromo- or p-methyl group; or $L^1$ represents a triphenylphosphonium-oxy group or an $-OCOCF_3$ group.

3. A process as claimed in claim 1 or claim 2, wherein the trivalent organophosphorus compound which is used to effect cyclisation is a phosphite or phosphoramide of the following formulae VII or VIII, or a phosphine of formula IX in combination with a phosphite of formula VII

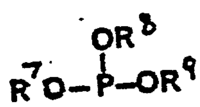

(VII)

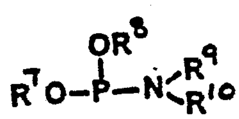

(VIII)

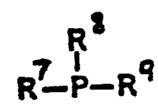

(IX)

in which formulae $R^7$, $R^8$, $R^9$ and $R^{10}$, which may be the same or different, each represents a straight or branched chain alkyl group having from 1 to 6 carbon atoms or a phenyl group which may be unsubstituted or substituted, for example, by a methyl group; or a cyclic trialkyl phosphite, each alkyl moiety having from 1 to 4 carbon atoms, for example, of formula X

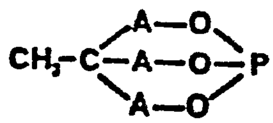 (X)

in which each group A denotes an alkylene group having from 1 to 4 carbon atoms; or a catechol phosphite or catechol dimer phosphite, for example, of the following formula XI or XII, respectively:

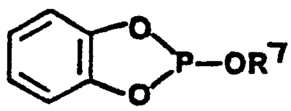 (XI) 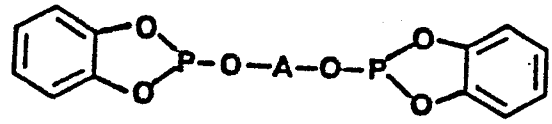 (XII)

in which $R^7$ and A are defined as above.

4. A process as claimed in any one of claims 1 to 3, wherein $R^1$ and $R^2$ both represent hydrogen atoms.

5. A process as claimed in any one of claims 1 to 3, wherein $R^1$ and $R^2$ both represent methyl groups, or one represents a hydrogen atom and the other represents a methyl group or a 2-aminoethyl group.

6. A process as claimed in any one of claims 1 to 3, wherein

(i) n represents the integer 3 and $R^1$ and $R^2$ both represent hydrogen atoms;

(ii) n represents the integer 3, $R^1$ represents a hydrogen atom and $R^2$ represents a methyl group;

(iii) n represents the integer 3, and $R^1$ and $R^2$ both represent methyl groups; or

(iv) n represents the integer 3, $R^1$ represents a hydrogen atom and $R^2$ represents a 2-aminoethyl group.

7. A process as claimed in any one of claims 1 to 6,

wherein a protected carboxy group -COOR is an esterified carboxy group that can be converted by hydrolysis, by photolysis, by oxidation, by reduction or by esterase action to give the free acid of formula I or a carboxylate, for example the group R is a phenyl group or a methyl group substituted by one or more unsubstituted or substituted phenyl groups; and wherein a phenyl group, either as R or as a substituent of a methyl group, is optionally subsituted by one or more substituents selected from methoxy and nitro groups and halogen atoms, for example, R represents a benzyl, nitrobenzyl, methoxybenzyl, dimethoxybenzyl, benzhydryl or trityl group; or R represents a phenoxyethyl or trichloroethyl group; or R represents a trialkysilyl or trialkylsilyalkyl group in which each alkyl moiety has from 1 to 4 carbon atoms; or R represents a phthalidyl group; or R represents a methyl or ethyl group optionally subsituted by an acyl or acyloxy group, by an alkoxycarbonyloxy group, by an aminoalkanoyloxy group or by an optionally subsituted amino group, for example, R represents an acyloxymethyl or acyloxyethyl group having from 2 to 12 carbon atoms in the acyl moiety, an aminoalkanoyloxymethyl group having from 2 to 12 carbon atoms in the alkanoyl moiety, a 1'-(alkoxycarbonyloxy)ethyl group, or an optionally substituted 2-aminoethyl group, especially a glycyloxymethyl, L-valinyloxymethyl or L-leucyloxymethyl group, a 1'-(methoxycarbonyloxy)ethyl group, a 2-diethylaminoethyl or 2-(1-morpholino)ethyl group, a pivaloyloxymethyl, ethoxycarbonyloxymethyl, acetylmethyl, acetoxymethyl, 1'-(acetoxy)ethyl, 1'-(acetyl)ethyl or 1'-(ethoxycarbonyloxy)ethyl group; or R represents a 5-methyl-dioxalen-2-on-4-yl-methyl group.

BAD ORIGINAL

in which $R^4$ is as defined in claim 1 and $R^{11}$ represents a group that can be displaced by the azetidinone nitrogen in the compound of formula XIII.

16. A process for the production of a compound of formula XIII, which comprises reacting a compound of formula XV

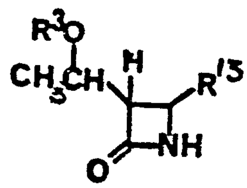

(XV)

in which $R^3$ is as defined in claim 1 and preferably represents a hydroxy protecting group, especially a silyl ether group, and $R^{13}$ represents a group that can be replaced in a nucleophilic displacement reaction with a compound of formula XVI

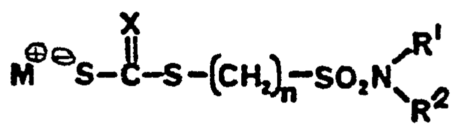

(XVI)

in which X, $R^1$, $R^2$ and n are as defined in claim 1, and M represents an alkali metal or alkaline earth metal atom, or an ammonium group that is unsubstituted or subsituted, for example, by one to four groups selected from alkyl groups having from 1 to 4 carbon atoms.

0211236

European Patent Office

**PARTIAL EUROPEAN SEARCH REPORT**
which under Rule 45 of the European Patent Convention shall be considered, for the purposes of subsequent proceedings, as the European search report

Application number
EP 86 10 8983

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 127 847 (HOECHST)<br>* Claims * | 1,12-14 | C 07 D 499/00<br>C 07 D 205/08<br>A 61 K 31/43//<br>C 07 F 7/18 |
| Y | CHEMICAL & PHARMACEUTICAL BULLETIN, vol. 28, no. 11, November 1980, pages 3258-3264; Pharmaceutical Society of Japan, JP<br>S. OIDA et al.: "2-(Alkylthio)penem-3-carboxylic acids. II. Chemical manipulation of penem side chains"<br>* Page 3259, formula 3f',h',l' * | 1 | |

TECHNICAL FIELDS SEARCHED (Int. Cl.4)

C 07 D 499/00
C 07 D 205/00
A 61 K 31/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-14,16,17
Claims searched incompletely:
Claims not searched: 15
Reason for the limitation of the search:

Method for treatment of the human or animal body by surgery or therapy (see art. 52(4) of the Europen Patent Convention).

| Place of search | Date of completion of the search | Examiner |
|---|---|---|
| The Hague | 19-09-1986 | CHOULY |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document